(19) Europäisches Patentamt
European Patent Office
Office européen des brevets

(11) Veröffentlichungsnummer: **0 315 014 B1**

(12) **EUROPÄISCHE PATENTSCHRIFT**

(45) Veröffentlichungstag der Patentschrift: **12.01.94**

(21) Anmeldenummer: **88117693.7**

(22) Anmeldetag: **25.10.88**

(51) Int. Cl.⁵: **C09K 19/20**, C09K 19/30, C09K 19/42, //C07C69/753, C07C69/76,C07D239/26

# (54) Halogenierte Benzolderivate.

(30) Priorität: **06.11.87 CH 4344/87**
**18.08.88 CH 3080/88**

(43) Veröffentlichungstag der Anmeldung:
**10.05.89 Patentblatt 89/19**

(45) Bekanntmachung des Hinweises auf die Patenterteilung:
**12.01.94 Patentblatt 94/02**

(84) Benannte Vertragsstaaten:
**CH DE FR GB IT LI NL**

(56) Entgegenhaltungen:
**DE-A- 3 608 764**
**GB-A- 2 179 039**
**US-A- 4 621 901**
**US-A- 4 701 547**

**PATENT ABSTRACTS OF JAPAN, unexamined applications, Sektion C, Band 9, Nr. 52, 6. März 1985 THE PATENT OFFICE JAPANESE GOVERNMENT Seite 165 C 269**

(73) Patentinhaber: **F. HOFFMANN-LA ROCHE AG**
**Postfach 3255**
**CH-4002 Basel(CH)**

(72) Erfinder: **Boller, Arthur, Dr.**
**Benkenstrasse 65**
**CH-4102 Binningen(CH)**
Erfinder: **Buchecker, Richard, Dr.**
**Felsenstrasse 10**
**CH-8008 Zürich(CH)**
Erfinder: **Schadt, Martin, Dr.**
**Liestalerstrasse 77**
**CH-4411 Seltisberg(CH)**
Erfinder: **Villiger, Alois, Dr.**
**Im Ettingerhof 5**
**CH-4055 Basel(CH)**

(74) Vertreter: **Cottong, Norbert A. et al**
**Grenzacherstrasse 124**
**Postfach 3255**
**CH-4002 Basel (CH)**

EP 0 315 014 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Halogenobenzolderivate, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen). Gast/Wirt Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmateralien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollten sie bei üblichen Betriebstemperaturen von etwa -30 °C bis etwa +80 °C, insbesondere von etwa -20 °C bis etwa +60 °C eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Verbindungen mit hoher optischer Anisotropie besteht in letzter Zeit ein vermehrtes Interesse an Materialien mit niedriger optischer Anisotropie, insbesondere für aktiv adressierte Flüssigkristallanzeigen. z.B. für TFT-Anwendungen ("thin film transistor" = Dünnfilmtransistor) in Fernsehgeräten, wobei jedoch Effekte, wie das Auftreten von hochgeordneten smektischen Phasen oder eine Erhöhung der Schwellenspannung und der Ansprechzeiten, die bei solchen Materialien häufig beobachtet werden, möglichst vermieden werden sollten.

Da Flüssigkristalle in der Regel als Mischungen mehrerer Komponenten zur Anwendung gelangen, ist es wichtig, dass die Komponenten untereinander gut mischbar sind.

Gegenstand der Erfindung sind die Halogenobenzolderivate der allgemeinen Formel

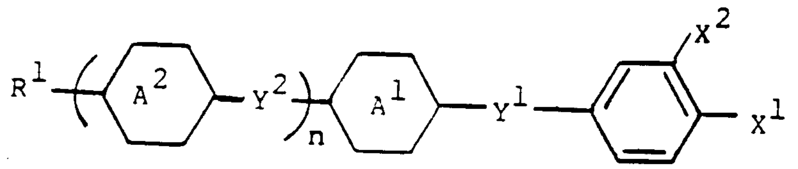

I

worin $X^1$ Fluor oder Chlor und $X^2$ Wasserstoff, Fluor oder Chlor bezeichnen; $R^1$ 3E-Alkenyl mit 4-15 Kohlenstoffatomen, 4-Alkenyl mit 5-15 Kohlenstoffatomen, 2E-Alkenyloxy mit 3-14 Kohlenstoffatomen oder 3-Alkenyloxy mit 4-14 Kohlenstoffatomen bedeutet; n für die Zahl 0 oder 1 steht; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 CH-Gruppen durch Stickstoff ersetzt sind, darstellen; mit der Massgabe, dass $X^2$ Fluor oder Chlor bezeichnet und/oder $Y^1$ oder $Y^2$ $-CH_2O-$ oder $-OCH_2$ bezeichnet, wenn zugleich $X^1$ Fluor und $R^1$ 3E-Alkenyl bedeuten.

Die erfindungsgemässen Verbindungen besitzen in der Regel eine positive dielektrische Anisotropie. Obwohl sie im Vergleich zu Cyanoverbindungen relativ wenig polar sind, ergeben sie erstaunlich tiefe Schwellenspannungen, die oft niedriger sind als jene von Cyanoverbindungen. Ferner besitzen sie eine hohe Stabilität und eine geringere elektrische Leitfähigkeit als die Cyanoverbindungen.

Ueberraschenderweise ergeben die erfindungsgemässen Verbindungen neben den tiefen Schwellenspannungen zugleich kurze Ansprechzeiten und hochgeordnete smektische Phasen werden ganz oder zumindest weitgehend unterdrückt, wobei oft auch die Schmelzpunkte herabgesetzt werden.

Die erfindungsgemässen Verbindungen eignen sich daher insbesondere als Komponenten nematischer und cholesterischer Gemische. Die bicyclischen Verbindungen der Formel I sind vor allem als Dotierstoffe zur Erzielung besonders niedriger Schwellenspannungen, Ansprechzeiten und Schmelzpunkte geeignet. Die tricyclischen Verbindungen der Formel I besitzen im allgemeinen einen breiten nematischen oder cholesterischen Mesophasebereich und eignen sich zur Erzielung breiter Mesophasebereiche in Gemischen, wobei zugleich die Schwellenspannungen und Ansprechzeiten erstaunlich niedrig bleiben.

Dank der guten Löslichkeit der Verbindungen der Formel I untereinander und in bekannten Flüssigkristallen können im allgemeinen vergleichsweise hohe Konzentrationen verwendet und somit die Zahl der Komponenten in flüssigkristallinen Gemischen verringert werden. Dies gilt inbesondere auch für die tricyclischen Verbindungen der Formel I.

Die optische Anisotropie der Verbindungen der Formel I kann je nach Wahl der Ringe $A^1$ und $A^2$ in einem breiten Bereich variieren, wobei gesättigte Ringe zu tiefen und aromatische Ringe zu hohen Werten der optischen Anisotropie führen. Die oben erwähnen Vorteile gelten im ganzen Bereich und werden insbesondere auch für die Verbindungen mit niedriger optischer Anisotropie gefunden.

Die Ausdrücke "3E-Alkenyl", "4-Alkenyl", "2E-Alkenyloxy" und "3-Alkenyloxy" in Formel I umfassen geradkettige und verzweigte Reste. Bevorzugt sind im allgemeinen die geradkettigen Reste, wie 3-Butenyl, 3E-Pentenyl, 3E-Hexenyl, 3E-Heptenyl, 3E-Octenyl, 3E-Nonenyl, 3E-Decenyl, 4-Pentenyl, 4-Hexenyl, 4-Heptenyl, 4-Octenyl, 4-Nonenyl, 4-Decenyl, Allyloxy, 2E-Butenyloxy, 2E-Pentenyloxy, 2E-Hexenyloxy, 2E-Heptenyloxy, 2E-Octenyloxy, 2E-Nonenyloxy, 3-Butenyloxy, 3-Pentenyloxy, 3-Hexenyloxy, 3-Heptenyloxy, 3-Octenyloxy, 3-Nonenyloxy und dergleichen. Verbindungen mit verzweigtem optisch aktivem Rest $R^1$ wie z.B. 5-Methyl-3E-heptenyl und 6-Methyl-4-octenyl sind vor allem als chirale Dotierstoffe und/oder cholesterische Flüssigkristalle von Interesse. Im Falle von 4-Alkenyl- und 3-Alkenyloxyresten $R^1$, die in E- oder Z-Form vorliegen können, ist im allgemeinen die Z-Form bevorzugt. Die 3E-Alkenylreste haben 4-15, die 4-Alkenylreste 5-15, die 2E-Alkenyloxyreste 3-14 und die 3-Alkenyloxyreste 4-14 Kohlenstoffatome. Im allgemeinen sind Alkenylreste mit bis zu 10 und Alkenyloxyreste mit bis zu 9 Kohlenstoffatomen bevorzugt. Besonders bevorzugte Reste $R^1$ sind 3-Butenyl, 3E-Pentenyl, 4-Pentenyl, Allyloxy und 3-Butenyloxy.

Der Ausdruck "substituiertes oder unsubstituiertes trans-1,4-Cyclohexylen, in welchem gegebenenfalls 2 nicht benachbarte $CH_2$-Gruppen durch Sauerstoff ersetzt sind," (im Rahmen der vorliegenden Erfindung auch als "gesättigter Ring" bezeichnet) umfasst insbesondere trans-1,4-Cyclohexylen und trans-m-Dioxan-2,5-diyl sowie Ringe, die mit in Flüssigkristallen üblichen Substituenten, wie Cyano, Methyl, Fluor oder Chlor, substituiert sind, beispielsweise 1-Cyano-trans-1,4-cyclohexylen oder 2-Methyl-trans-1,4-cyclohexylen.

Der Ausdruck "substituiertes oder unsubstituiertes 1,4-Phenylen, in welchem gegebenenfalls 1 oder 2 CH-Gruppen durch Stickstoff ersetzt sind," (im Rahmen der vorliegenden Erfindung auch als "aromatischer Ring" bezeichnet) umfasst insbesondere 1,4-Phenylen, Pyridin-2,5-diyl, Pyrazin-2,5-diyl und Pyrimidin-2,5-diyl, sowie Ringe, die mit in Flüssigkristallen üblichen Substituenten, wie Cyano, Methyl, Fluor oder Chlor substituiert sind, beispielsweise 2-Cyano-1,4-phenylen, 2-Fluor-1,4-phenylen, 2-Chlor-1,4-phenylen oder 2-Methyl-1,4-phenylen.

Im allgemeinen sind Verbindungen der Formel I mit unsubstituierten Ringen $A^1$ und $A^2$ bevorzugt. Gewünschtenfalls können jedoch durch Verwendung substituierter Ringe die dielektrische Anisotropie, die Mesophasenbereiche, die Löslichkeit und dergleichen modifiziert werden.

Formel I umfasst die Verbindungen der folgenden allgemeinen Formeln

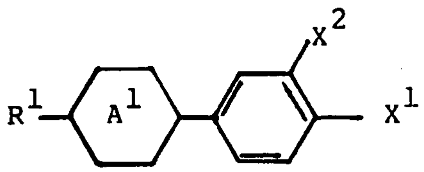

I - 1

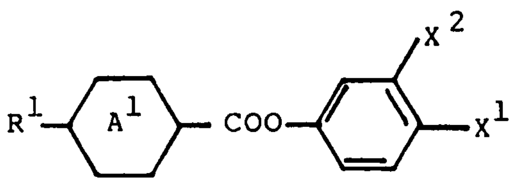

I - 2

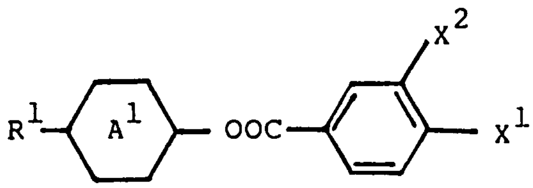

I - 3

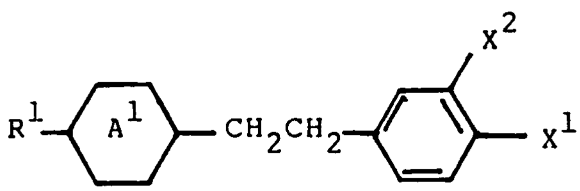

I - 4

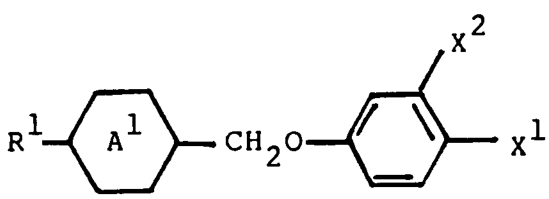

I - 5

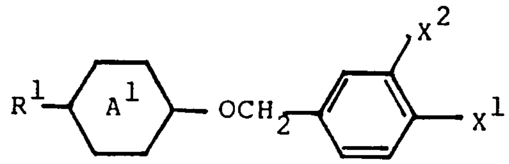

I - 6

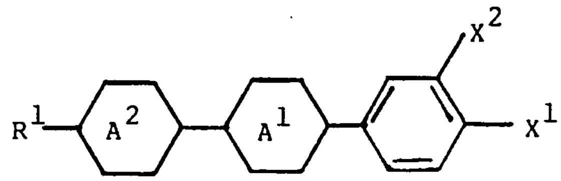

I-7

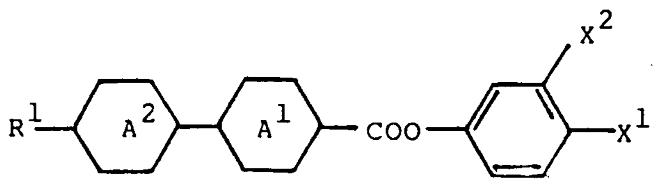

I-8

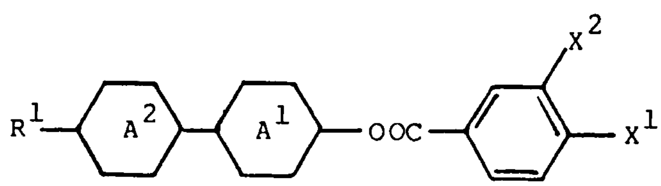

I-9

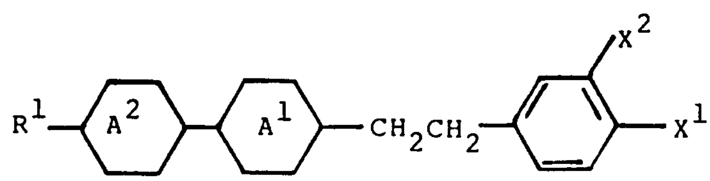

I-10

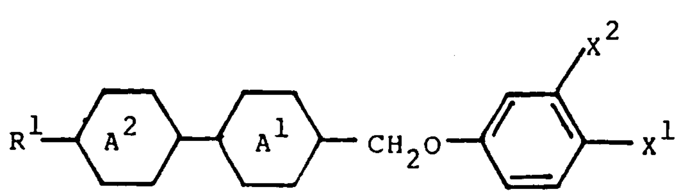

I-11

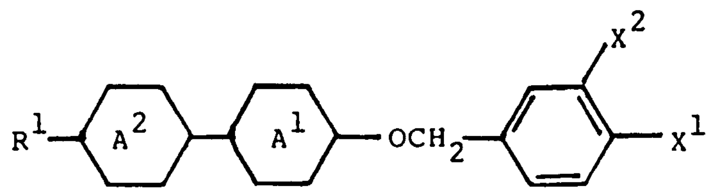

I-12

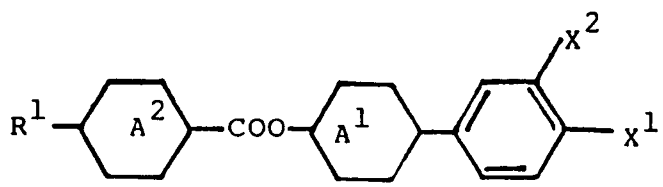

I-13

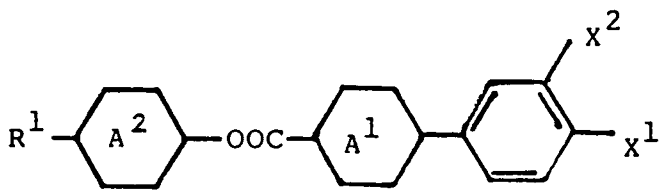

I-14

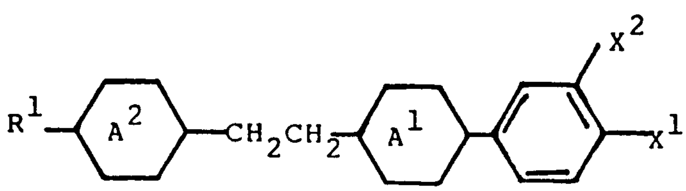

I-15

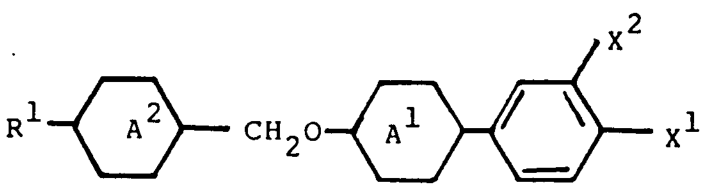

I-16

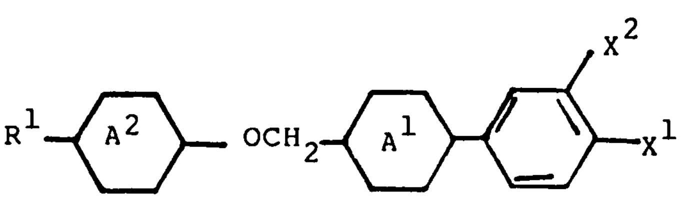

I-17

worin $R^1$, $X^1$, $X^2$ und die Ringe $A^1$ und $A^2$
jeweils die obigen Bedeutungen haben.

In obigen Formeln I, I-7 und I-8 bis I-17 steht Ring $A^2$ vorzugsweise für substituiertes oder unsubstituiertes trans-1,4-Cyclohexylen, für substituiertes oder unsubstituiertes 1,4-Phenylen oder, wenn Ring $A^1$ substituiertes oder unsubstituiertes trans-1,4-Cylohexylen oder substituiertes oder unsubstituiertes 1,4-Phenylen bedeutet, auch für Pyrimidin-2,5-diyl oder trans-m-Dioxan-2,5-diyl. Besonders bevorzugt sind im allgemeinen diejenigen Verbindungen, worin Ring $A^2$ für trans-1,4-Cyclohexylen steht. Eine für Ring $A^2$ gegebenenfalls vorhandene trans-m-Dioxan-2,5-diyl-Gruppeist vorzugsweise in 5-Position mit $R^1$ verknüpft; Ring $A^1$ bezeichnet in diesem Fall vorzugsweise trans-1,4-Cyclohexylen.

Ferner steht in obigen Formeln I und I-1 bis I-17 Ring $A^1$ jeweils vorzugsweise für substituiertes oder unsubstituiertes trans-1,4-Cyclohexylen, für substituiertes oder unsubstituiertes 1,4-Phenylen oder für Pyridin-2,5-diyl, Pyrimidin-2,5-diyl oder Pyrazin-2,5 diyl. Von den Verbindungen der Formeln I, I-7 und I-8 bis I-17 sind im allgemeinen diejenigen besonders bevorzugt, worin Ring $A^1$ eine der genannten, bevorzugten Bedeutungen hat, und insbesondere eine unsubstituierte Gruppe bezeichnet, und Ring $A^2$ zugleich trans-1,4-Cyclohexylen bedeutet.

Beispiele bevorzugter Gruppen von Verbindungen der Formel I sind die Verbindungen der allgemeinen Formeln

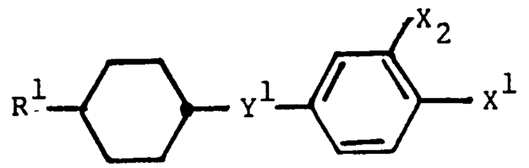

I-18

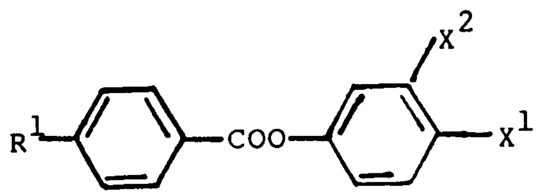

I-19

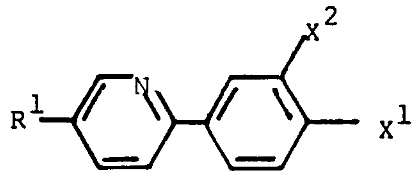

I-20

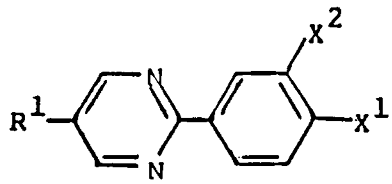

I-21

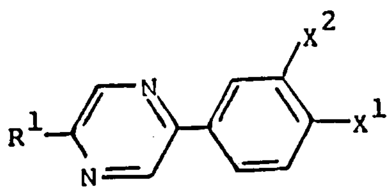

I-22

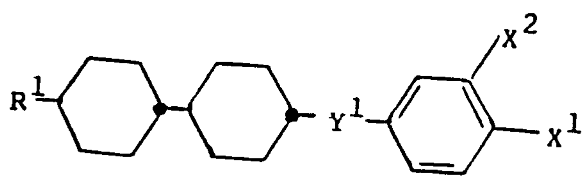

I-23

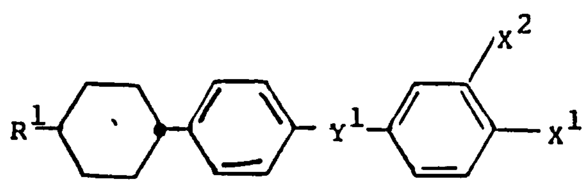

I-24

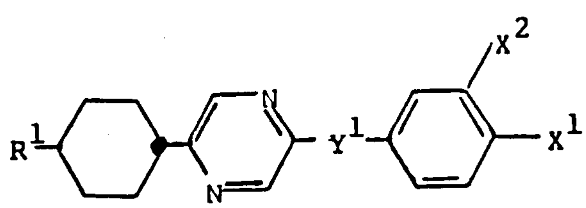

I-25

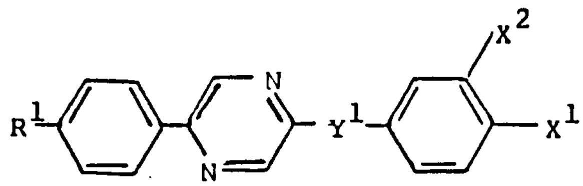

I-26

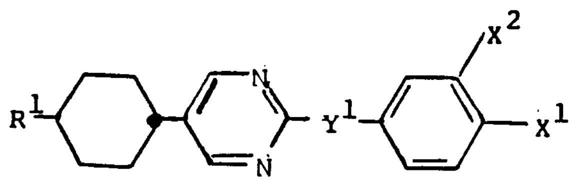

I-27

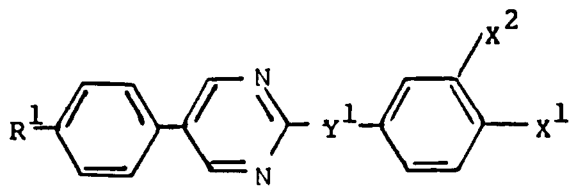

I-28

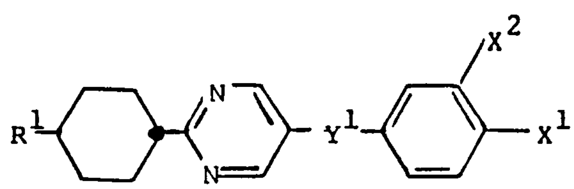

I-29

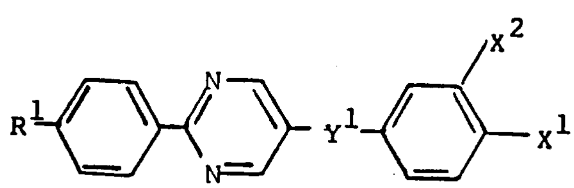

I-30

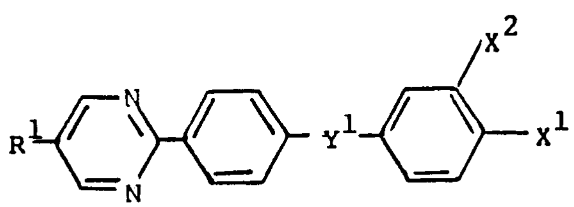

I-31

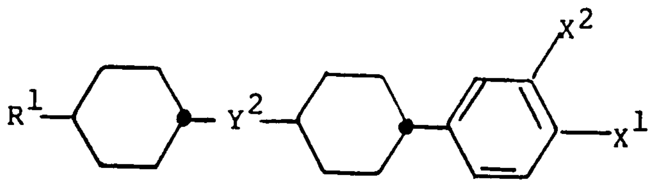

I-32

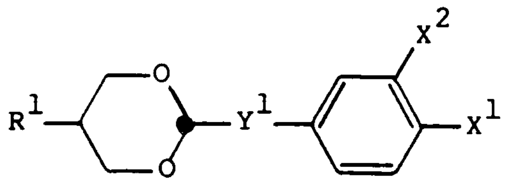

I-33

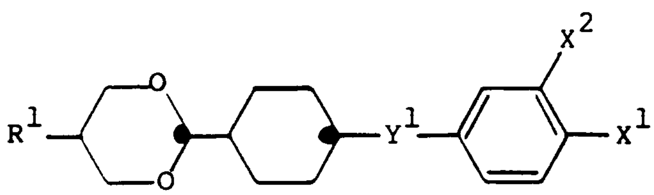

I-34

worin $R^1$, $X^1$, $X^2$, $Y^1$ und $Y^2$ die obigen Bedeutungen haben.

Von den Verbindungen der obigen Formeln I, I-1 bis I-32, I-33 und I-34 sind im allgemeinen diejenigen bevorzugt, worin jeweils $X^1$ Fluor und $X^2$ Wasserstoff bedeuten, oder $X^1$ und $X^2$ Fluor bedeuten, oder $X^1$ Chlor und $X^2$ Wasserstoff bedeuten. Ferner sind von den Verbindungen der Formeln I-18, 1-23 bis I-31, I-33 und I-34 im allgemeinen die jenigen bevorzugt. worin jeweils $Y^1$ eine einfache Kovalenzbindung oder -COO- bedeutet. Bevorzugte Reste $R^1$ in den obigen Formeln I und I-1 bis I-34 sind 3-Butenyl, 3E-Pentenyl und 4-Pentenyl.

Die Verbindungen der Formel I können in an sich bekannter Weise hergestellt werden, beispielsweise nach den in EP-A-122389, EP-A-167912 und EP-A-168683 beschriebenen Methoden.

Die Verbindungen der Formel I, worin $R^1$ 2E-Alkenyloxy oder 3-Alkenyloxy bedeutet, werden im allgemeinen am einfachsten dadurch erhalten, dass man die entsprechende Hydroxyverbindung mit 2E-Alkenylhalogenid oder 3-Alkenylhalogenid, vorzugsweise 2E-Alkenylbromid oder 2E-Alkenyljodid, 3-Alkenylbromid oder 3-Alkenyljodid veräthert. Die benötigten Hydroxyverbindungen sind bekannte oder Analoge bekannter Verbindungen.

Die Verbindungen der Formel I, worin $R^1$ 3E-Alkenyl oder 4-Alkenyl bedeutet, werden im allgemeinen dadurch erhalten, dass man den entsprechenden Propionaldehyd bzw. Butyraldehyd in Gegenwart einer starken Base mit Alkyl-triarylphosphoniumhalogenid,vorzugsweise mit Alkyl-triphenyl phosphoniumchlorid oder mit Alkyl-triphenylphosphoniumbromid umsetzt. Die Umsetzung kann unter den für Wittig-Reaktionen üblichen Bedingungen erfolgen. Die benötigten Propionaldehyde und Butyraldehyde können aus den entsprechenden Verbindungen mit formylsubstituiertem Ring (Derivate von Cyclohexancarboxaldehyd, Benzaldehyd etc.) oder den entsprechenden cyclischen Ketonen (Cyclohexanonderivate) durch sukzessive Kettenverlängerung via die Acetaldehyde erhalten werden. Die Kettenverlängerung kann z.B. jeweils durch Wittig-Reaktion mit Methoxymethyl-triarylphosphoniumhalogenid, vorzugsweise Methoxymethyl-triphenylphosphoniumchlorid, und anschliessende Hydrolyse (beispielsweise mit Essigsäure) erfolgen. Ferner kann eine Kettenverlängerung um 3 Kohlenstoffatome z.B. auch durch Wittig-Reaktion mit 2-(1,3-Dioxolan-2-yl)-äthyl-triarylphosphoniumhalogenid, vorzugsweise 2-(1,3-Dioxolan-2-yl)äthyl-triphenylphoshoniumbromid, anschliessende katalytische Hydrierung der C-C-Doppelbindung und Hydrolyse des Dioxolan-Ringes, z.B. mit Essigsäure, erfolgen. Letztere Methode hat zudem den Vorteile, dass vor der Hydrolyse des Dioxolan-Ringes gewünschtenfalls leicht andere funktionelle Gruppen im Molekül eingeführt oder umgesetzt werden können.

Wenn der zentrale Teil der Formel I eine Gruppe aufweist, die leicht durch abschliessende Verknüpfung gebildet werden kann, wie beispielsweise eine Estergruppe oder Methylenoxygruppe für $Y^1$ oder $Y^2$ oder einen Dioxanring für Ring $A^1$ oder $A^2$, kann es von Vorteil sein, das entsprechende Edukt (z.B. Carbonsäure,

Hydroxyverbindung, Halogenverbindung, 1,3-Diol, Aldehyd) enthaltend die Alkenylgruppe oder die Alkenyloxygruppe in analoger Weise zu den oben beschriebenen Methoden herzustellen. Anschliessend kann beispielsweise die erhaltene Carbonsäure mit einer geeigneten Hydroxyverbindung verestert werden (zu einer Verbindung der Formel I, worin $Y^1$ oder $Y^2$ -COO- bedeutet), oder die erhaltene Hydroxyverbindung mit einer geeigneten Carbonsäure oder einem geeigneten Carbonsäurechlorid verestert werden (zu einer Verbindung der Formel I, worin $Y^1$ oder $Y^2$ -OOC- bedeutet), oder eine erhaltene Hydroxyverbindung mit einer geeigneten Halogenverbindung (insbesondere einer Brom oder Jodverbindung) veräthert werden (zu einer Verbindung der Formel I, worin $Y^1$ oder $Y^2$ $-OCH_2-$ bedeutet), oder eine erhaltene Halogenverbindung (insbesondere eine Brom- oder Jodverbindung) mit einer geeigneten Hydroxyverbindung veräthert werden (zu einer Verbindung der Formel I, worin, $Y^1$ oder $Y^2$ $-CH_2O-$ bedeutet), oder ein erhaltenes 1,3-Diol mit einem geeigneten Aldehyd bzw. ein erhaltener Aldehyd mit einem geeigneten 1,3-Diol umgesetzt werden (zu einer Verbindung der Formel I, worin Ring $A^1$ oder $A^2$ trans-m-Dioxan-2,5-diyl bedeutet).

Die angegebenen Reaktionen können jeweils in an sich bekannter Weise erfolgen. Die benötigten Ausgangsmaterialien sind bekannt oder können in an sich bekannter Weise hergestellt werden. Die obigen Synthesevarianten und die Reaktionsbedingungen sind in den Synthesebeispielen weiter veranschaulicht.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Die Verbindungen der Formel I eignen sich insbesondere für nematische oder, sofern mindestens eine Komponente des Gemisches optisch aktiv ist, auch für cholesterische Gemische.

Die Verbindungen der Formel I, worin n für die Zahl 0 steht und $R^1$ 4-Alkenyl bedeutet, werden im allgemeinen mit Vorteil zusammen mit anderen nematischen oder cholesterischen Materialien, beispielsweise auch zusammen mit tricyclischen Verbindungen der Formel I verwendet. Die erfindungsgemässen Gemische enthalten daher vorzugsweise eine oder mehrere Verbindungen der Formel I und ein nematisches oder cholesterisches Trägermaterial.

Aufgrund der guten Löslichkeit der Verbindungen der Formel I und aufgrund ihrer guten Mischbarkeit untereinander kann ihr Anteil in den erfindungsgemässen Gemischen relativ hoch sein. Er liegt bei 1-50 Gew.-%, insbesondere bei 5-30 Gew.-% an Verbindungen der Formel I.

Vorzugsweise enthalten die erfindungsgemässen Gemische neben einer oder mehreren Verbindungen der Formel I eine oder mehrere Verbindungen aus der Gruppe der Verbindungen der allgemeinen Formel

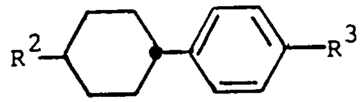

II

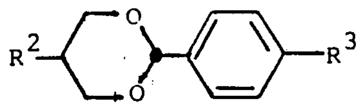

III

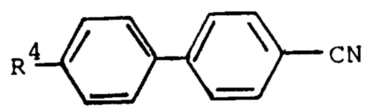

IV

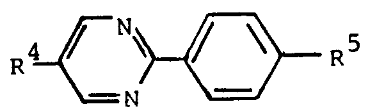

V

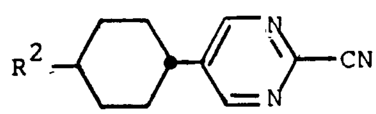

VI

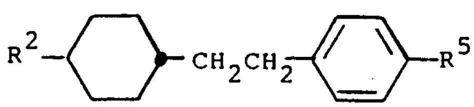

VII

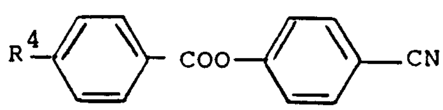

VIII

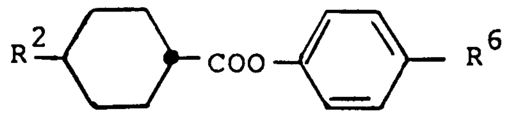

IX

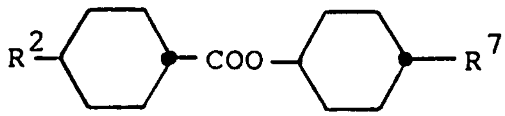

X

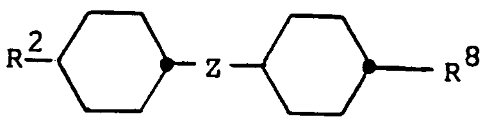

XI

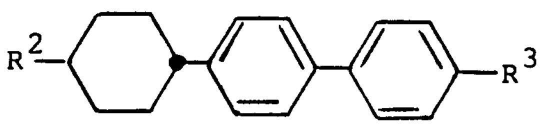

XII

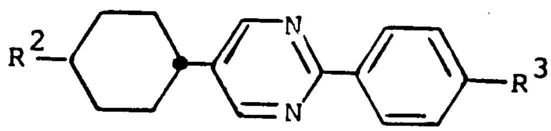

XIII

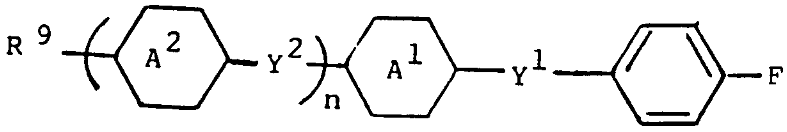

XIV

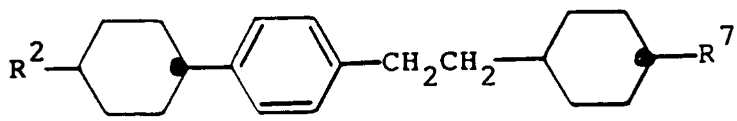

XV

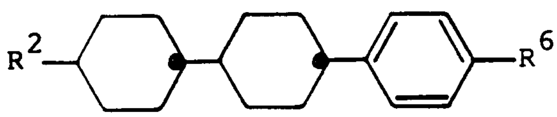

XVI

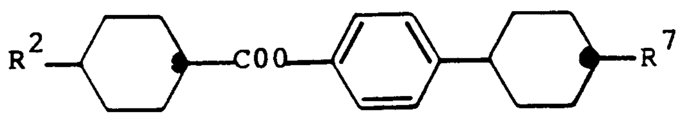

XVII

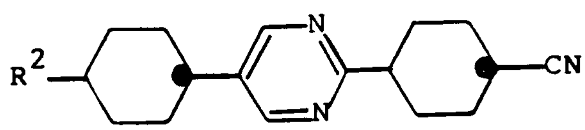

XVIII

worin $R^2$ und $R^7$ Alkyl, 1E-Alkenyl, 3E-Alkenyl oder 4-Alkenyl bedeuten; $R^3$ Cyano, -NCS, Alkyl, 1E-Alkenyl, 3E-Alkenyl, 4-Alkenyl, Alkoxy, 2E-Alkenyloxy oder 3-Alkenyloxy bedeutet; $R^4$ Alkyl, 3E-Alkenyl oder 4-Alkenyl bedeutet; $R^5$ Cyano, -NCS, Alkyl, 3E-Alkenyl, 4-Alkenyl, Alkoxy, 2E-Alkenyloxy oder 3-Alkenyloxy bedeutet; $R^6$ Cyano, Alkyl, 3E-Alkenyl, 4-Alkenyl, Alkoxy, 2E-Alkenyloxy oder 3-Alkenyloxy bedeutet; Z eine einfache Kovalenzbindung oder $-CH_2-CH_2-$ bedeutet; $R^8$ Cyano, Alkoxy, 2E-Alkenyloxy, 3-Alkenyloxy, Alkyl, 1E-Alkenyl, 3E-Alkenyl oder 4-Alkenyl bedeutet; $R^9$ Alkyl, 1E-Alkenyl oder, wenn eine der Gruppen $Y^1$ und $Y^2$ eine einfache Kovalenzbindung, -COO-, -OOC- oder $-CH_2CH_2-$ und die andere der Gruppen $Y^1$ und $Y^2$ eine einfache Kovalenzbindung bedeutet, auch 3E-Alkenyl bezeichnet; und n, $Y^1$, $Y^2$ und die Ringe $A^1$ und $A^2$ die obigen Bedeutungen haben.

Die Alkyl-, Alkenyl-, Alkoxy- und Alkenyloxyreste $R^2$-$R^9$ in den Formeln II-XIV sind vorzugsweise geradkettige Reste. Sie weisen vorzugsweise bis zu 12, besonders bevorzugt bis zu 7 Kohlenstoffatome auf.

Die Verbindungen der Formel XI, worin $R^2$ 1E-Alkenyl, 3E-Alkenyl oder 4-Alkenyl bedeutet sind neu. Sie können nach den in den Synthesebeispielen illustrierten Methoden hergestellt werden.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I und der neuen Verbindungen der Formel XI sowie flüssigkristalline Gemische enthaltend diese Verbindungen werden durch die folgenden Beispiele weiter veranschaulicht. C bedeutet eine kristalline, $S_B$ eine smektisch B, N eine nematische und I die isotrope Phase. $V_{10}$ und $V_{50}$ bezeichnen die Spannung für 10% bzw. 50% Transmission, $p = (V_{50}-V_{10})/V_{10}$ ist ein Mass für die Steilheit der Transmissionskurve, $t_{on}$ und $t_{off}$ bezeichnen die Einschaltzeit bzw. die Ausschaltzeit und $\Delta n$ bezeichnet die optische Anisotropie.

Beispiel 1

a) Eine Lösung von 1,71 g trans-4-[trans-4-(4-Pentenyl)cyclohexyl]cyclohexancarbonitril (hergestellt nach Beispiel 2) in 30 ml Diäthylenglykol wurde mit 3,11 g Kaliumhydroxid versetzt und 3 Stunden bei 130 °C gerührt. Dann wurde das Gemisch auf Eiswasser gegossen, mit 25-prozentiger Salzsäure angesäuert und dreimal mit Diäthyläther extrahiert. Die organischen Phasen wurden vereinigt, dreimal mit Wasser gewaschen, über Magnesiumsulfat getrocknet und eingedampft. Umkristallisation des braunen, kristallinen Rückstandes (1,74 g) aus 30 ml Hexan ergab 996 mg trans-4-[trans-4-(4-Pentenyl)cyclohexyl]-cyclohexancarbonsäure als gelbliche Kristalle.

b) Eine Lösung von 996 mg trans-4-[trans-4-(4-Pentenyl)cyclohexyl]cyclohexancarbonsäure in 80 ml Methylenchlorid wurde mit 752 mg p-Fluorphenol, 61 mg 4-(Dimethylamino)pyridin und 1,03 g Dicyclohexylcarbodiimid versetzt und 15 Stunden bei Raumtemperatur gerührt. Anschliessend wurde das Reaktionsgemisch filtriert. Das Filtrat wurde eingedampft und der erhaltene Rückstand an Kieselgel mit Aethylacetat/Petroläther (Vol. 3:97) chromatographisch gereinigt. Kristallisation des erhaltenen Produktes (1,24 g) aus 40 ml Hexan ergab 757 mg reinen trans-4-[trans-4-(4-Pentenyl)cyclohexyl]-cyclohexancarbonsäure -4-fluorphenylester mit Smp. (C-N) 70,3 °C und Klp. (N-I) 158,7 °C.

In analoger Weise können folgende Verbindungen hergestellt werden:
trans-4-(4-Pentenyl)cyclohexancarbonsäure-4-fluorphenylester, Smp. 31,3 °C;
trans-4-(4-Pentenyl)cyclohexancarbonsäure-3,4-difluorphenylester, Smp. 12,4 °C;
trans-4-(4-Pentenyl)cyclohexancarbonsäure 4-chlorphenylester;
4-(4-Pentenyl)benzoesäure-4-fluorphenylester;
4-(4-Pentenyl)benzoesäure-3,4-difluorphenylester;
4-(3-Butenyloxy)benzoesäure-4-fluorphenylester, Smp. 65 °C;
4-(3-Butenyloxy)benzoesäure-3,4-difluorphenylester, Smp. 47,5 °C;
4-(3-Butenyloxy)benzoesäure-4-chlorphenylester;
trans-4-[trans-4-(4-Pentenyl)cyclohexyl]cyclohexancarbonsäure-3,4-difluorphenylester;
trans-4-[trans-4-(4-Pentenyl)cyclohexyl]cyclohexancarbonsäure-4-chlorphenylester;
trans-4-[trans-4-(4-Pentenyl)cyclohexyl]cyclohexancarbonsäure-3-chlor-4-fluorphenylester;
trans-4-[trans-4-(4-Pentenyl)cyclohexyl]cyclohexancarbonsäure-3-fluor-4-chlorphenylester;
4-[trans-4-(4-Pentenyl)cyclohexyl]benzoesäure-4-fluorphenylester;
4-[trans-4-(4-Pentenyl)cyclohexyl]benzoesäure-3,4-difluorphenylester;
5-[trans-4 (4-Pentenyl)cyclohexyl]-2-pyrimidincarbonsäure-4-fluorphenylester;
2-[trans-4-(4-Pentenyl)cyclohexyl]-5-pyrimidincarbonsäure-4-fluorphenylester;
4-[5-(4-Pentenyl)-2-pyrimidinyl]benzoesäure-4-fluorphenylester;
4-[2-(4-Pentenyl)-5-pyrimidinyl]benzoesäure-4-fluorphenylester;
trans-4-(4Z-Hexenyl)cyclohexancarbonsäure-4-fluorphenylester;
trans-4-(4Z-Hexenyl)cyclohexancarbonsäure-3,4-difluorphenylester;
4-(3Z-Pentenyloxy)benzoesäure-4-fluorphenylester;
trans-4-[trans-4-(4Z-Hexenyl)cyclohexyl]cyclohexancarbonsäure-4-fluorphenylester;
4-[trans-4-(4Z-Hexenyl)cyclohexyl]benzoesäure-4-fluorphenylester;
trans-4-(3-Butenyl)cyclohexancarbonsäure-3,4-difluorphenylester, Smp. 42,0 °C;
trans-4-(3-Butenyl)cyclohexancarbonsäure-4-chlorphenylester;
4-Allyoxybenzoesäure-4-fluorphenylester;
4-Allyloxybenzoesäure-3,4-difluorphenylester;
4-Allyloxybenzoesäure-4-chlorphenylester;
trans-4-[trans-4-(3-Butenyl)cyclohexyl]cyclohexancarbonsäure-3,4-difluorphenylester, Smp. (C-N) 55,1 °C, Klp. (N-I) 153,6 °C;
trans-4-[trans-4-(3-Butenyl)cyclohexyl]cyclohexancarbonsäure-4-chlorphenylester, Smp. (C-N) 78,3 °C, Klp. (N-I) 213,5 °C;
trans-4-[trans-4-(3-Butenyl)cyclohexyl]cyclohexancarbonsäure-3-chlor-4-fluorphenylester;
trans-4-[trans-4-(3-Butenyl)cyclohexyl]cyclohexancarbonsäure-3-fluor-4-chlorphenylester;
4-[trans-4-(3-Butenyl)cyclohexyl]benzoesäure-3,4-difluorphenylester;
trans-4-(3E-Pentenyl)cyclohexancarbonsäure-3,4-difluorphenylester;
4-(2E-Butenyloxy)benzoesäure-4-fluorphenylester.

Beispiel 2

a) 140,9 g 2-(1,3-Dioxolan-2-yl)äthyl-triphenylphosphoniumbromid wurden unter Stickstoffbegasung in 4,5 l Tetrahydrofuran suspendiert und die Suspension bei 0°C innert 5 Minuten mit 36,8 g Kalium-tert.butylat versetzt. Die orange Suspension wurde noch 1 Stunde bei Raumtemperatur gerührt und innert 5 Minuten mit 46,1 g 4-(4-Oxocyclohexyl)benzamid versetzt. Das Reaktionsgemisch wurde noch 4,5 Stunden bei Raumtemperatur gerührt und dann im Vakuum eingeengt. Die erhaltenen, gelblichen Kristalle (212,5 g) wurden mit 1,2 l Diäthyläther versetzt. Das Gemisch wurde 30 Minuten bei Raumtemperatur gerührt und dann genutscht. Der Rückstand wurde mit Diäthyläther gewaschen und dann in 700 ml Wasser aufgeschlämmt. Das Gemisch wurde 15 Minuten gerührt und dann genutscht. Der Rückstand wurde mit Wasser gewaschen und in 600 ml Dioxan heiss gelöst. Die Lösung wurde auf Raumtemperatur abkühlen gelassen. Die ausgefallenen Kristalle wurden abgenutscht, mit wenig Dioxan und Hexan gewaschen und im Vakuum bei 60°C getrocknet, wobei 39,2 g kristallines 4-[4-[2-(1,3-Dioxolan-2-yl)-äthyliden]cyclohexyl]benzamid mit Smp. 209-212°C erhalten wurden. Aufarbeitung der Mutterlauge (72,6 g) ergab weitere 7,7 g Produkt und 64,2 g zweite Mutterlauge.

b) Ein Gemisch von 500 mg 4-[4-[2-(1,3-Dioxolan-2-yl)äthyliden]cyclohexyl]benzamid und 20 ml Dioxan/Triäthylamin (Vol. 9:1) wurde mittels 500 mg 10-prozentiger Platin-Kohle 2 Stunden hydriert. Dann wurde das Reaktionsgemisch filtriert und das Filtrat eingedampft. Umkristallisation des Eindampfrückstandes aus 40 ml Dioxan ergab 230 mg 4-[trans-4-[2-(1,3-Dioxolan-2-yl)äthyl]cyclohexyl]benzamid als farblose Kristalle.

c) 27 g 4-[trans-4-[2-(1,3-Dioxolan-2-yl)äthyl]cyclohexyl]benzamid wurden mit 5 g 5-prozentiger Ruthenium Kohle in Dioxan bei 120°C und 40 bar Wasserstoff 8 Stunden hydriert. Das nach Filtration und Waschen mit Tetrahydrofuran erhaltene Rohprodukt (25 g) enthielt 69% cis-Isomer und 25% trans-Isomer von 4-[trans-4-[2-(1,3-Dioxolan -2-yl)äthyl]cyclohexyl]cyclohexancarboxamid.

d) 42 g rohes 4-[trans-4-[2-(1,3-Dioxolan -2-yl)äthyl]cyclohexyl]cyclohexancarboxamid wurden unter Argonbegasung in 500 ml Aethylenglykol aufgeschlämmt und dann mit 19 g festem Kaliumhydroxid versetzt. Das Gemisch wurde unter Rühren 5 Stunden auf 180°C (Badtemperatur) erhitzt. Nach dem Abkühlen wurde das Reaktionsgemisch auf 500 ml Wasser gegossen, mit 10-prozentiger Salzsäure auf pH ca. 3 angesäuert und dreimal mit je 300 ml Methylenchlorid extrahiert. Die vereinigten organischen Phasen wurden einmal mit 500 ml 1-prozentiger Salzsäure und zweimal mit je 500 ml Wasser gewaschen, über Magnesiumsulfat getrocknet, filtriert und eingedampft. Das dunkelbraune Rohprodukt (41 g) wurde an Kieselgel mit Aethylacetat chromatographisch gereinigt. Kristallisation des erhaltenen Produktes (36 g) aus Aceton ergab 13,8 g reine trans-4-[trans-4-[2-(1,3-Dioxolan -2-yl)äthyl]cyclohexyl]-cyclohexancarbonsäure. Aufarbeitung der Mutterlauge ergab weitere 2,5 g reines Produkt.

e) 18 g trans-4-[trans-4-[2-(1,3-Dioxolan -2-yl)äthyl]cyclohexyl]cyclohexancarbonsäure wurden unter Argonbegasung in 600 ml Chloroform gelöst und die Lösung bei 0°C unter Rühren innert 3 Minuten tropfenweise mit einer Lösung von 7,2 ml Chlorameisensäureäthylester in 40 ml Chloroform versetzt. Die Reaktionslösung wurde noch 30 Minuten gerührt. Dann wurde während 10 Minuten Ammoniak-Gas in die Lösung eingeleitet. Das Gemisch wurde noch 30 Minuten bei 0°C gerührt und dann zweimal mit je 300 ml Wasser extrahiert. Die wässrigen Phasen wurden mit je 100 ml Chloroform nachextrahiert. Die vereinigten organischen Phasen wurden über Magnesiumsulfat getrocknet, filtriert und eingedampft. Umkristallisation des erhaltenen, braunen kristallinen Rohproduktes (19 g) aus 800 ml Methylenchlorid ergab 13 g trans-4-[trans-4-[2-(1,3-Dioxolan -2-yl)äthyl]cyclohexyl]cyclohexancarboxamid als hellbraune Kristalle.

f) 2,1 g trans-4-[trans-4-[2-(1,3-Dioxolan -2-yl)äthyl]cyclohexyl]cyclohexancarboxamid wurden unter Argonbegasung in 60 ml Dimethylformamid aufgeschlämmt. Die Suspension wurde mit 1,32 ml Pyridin und 0,898 ml Methansulfochlorid versetzt und 1,5 Stunden bei 60°C (Badtemperatur) gerührt. Anschliessend wurde die Reaktionslösung in Methylenchlorid und 10-prozentiger Salzsäure verteilt. Die wässrige Phase wurde zweimal mit je 100 ml Methylenchlorid extrahiert. Die organischen Phasen wurden zweimal mit je 100 ml Wasser gewaschen, über Magnesiumsulfat getrocknet, filtriert und eingeengt. Hierbei wurden 2,5 g trans-4-[trans-4-[2-(1,3-Dioxolan -2-yl)äthyl]cyclohexyl]cyclohexancarbonitril als gelbe Kristalle in einer Reinheit von 96% erhalten.

g) 3,0 g rohes trans-4-[trans-4-[2-(1,3-Dioxolan -2-yl)äthyl]cyclohexyl]cyclohexancarbonitril wurden unter Argonbegasung in 50 ml Wasser, 25 ml Eisessig und 10 ml Dioxan aufgeschlämmt und 1 Stunde bei 100°C gerührt. Danach wurde die Reaktionslösung mit 100 ml Wasser versetzt. Die wässrige Phase wurde abgetrennt und dreimal mit je 100 ml Diäthylather extrahiert. Die organischen Phasen wurden mit 100 ml verdünnter Natriumhydrogencarbonat-Lösung und mit 100 ml Wasser gewaschen, über Magnesiumsulfat getrocknet, filtriert und eingeengt. Umkristallisation der erhaltenen gelben Kristalle (2,25 g) aus

60 ml Hexan ergab 1,98 g 3-[trans-4-(trans-4-Cyanocyclohexyl)cyclohexyl]propionaldehyd als farblose Kristalle.

h) 4,11 g Methoxymethyl-triphenylphosphoniumchlorid wurden unter Argonbegasung in 60 ml tert.Butylmethyläther aufgeschlämmt und bei Raumtemperatur innert 2 Minuten mit 1,26 g Kalium-tert.butylat versetzt. Die Suspension wurde noch 1 Stunde bei Raumtemperatur gerührt, dann auf 0 °C gekühlt und innert 5 Minuten tropfenweise mit einer Lösung von 1,98 g 3-[trans-4-(trans 4-Cyanocyclohexyl)cyclohexyl]propionaldehyd in 25 ml tert.Butylmethyläther versetzt. Das Reaktionsgemisch wurde noch 45 Minuten bei 0 °C gerührt, dann mit 100 ml Wasser verdünnt und dreimal mit je 100 ml Diäthyläther extrahiert. Die organischen Phasen wurden zweimal mit je 100 ml Wasser gewaschen, über Magnesiumsulfat getrocknet, filtriert und eingeengt. Chromatographische Reinigung des Rohprodukts (5,1 g) an Kieselgel bei 0,5 bar mit Aethylacetat/Petroläther (Vol. 5:95) ergab 2,0 g trans-4-[trans-4-(4-Methoxy -3-butenyl)cyclohexyl]cyclohexancarbonitril als farbloses milchiges Oel.

i) 1,65 g trans-4-[trans-4-(4-Methoxy -3-butenyl)cyclohexyl]cyclohexancarbonitril wurden unter Argonbegasung in 50 ml Wasser, 25 ml Eisessig und 12 ml Doxan aufgeschlämmt. Die Suspension wurde 2 Stunden bei 80 °C (Badtemperatur) gerührt und dann mit 50 ml Wasser verdünnt. Die wässrige Phase wurde abgetrennt und dreimal mit je 100 ml Diathyläther extrahiert. Die organischen Phasen wurden zweimal mit je 100 ml Wasser, dann mit 100 ml gesättigter Natriumhydrogencarbonat-Lösung und nochmals mit 100 ml Wasser gewaschen, dann über Magnesiumsulfat getrocknet, filtriert und eingeengt. Hierbei wurden 1,5 g 4-[trans-4-(trans-4-Cyanocyclohexyl)cyclohexyl]butyraldehyd als farblose Kristalle erhalten.

j) 3,28 g Methyltriphenylphosphoniumbromid wurden unter Argonbegasung in 40 ml tert.Butylmethyläther aufgeschlämmt. Die Suspension wurde bei Raumtemperatur innert 1 Minute mit 962 mg Kalium-tert.butylat versetzt und 1 Stunde gerührt. Anschliessend wurde das Gemisch auf 0 °C gekühlt, innert 3 Minuten tropfenweise mit einer Lösung von 1,5 g 4-[trans-4-(trans-4-Cyanocyclohexyl)cyclohexyl]-butyraldehyd in 20 ml tert.Butylmethyläther versetzt und noch 45 Minuten bei 0 °C gerührt. Danach wurde das Reaktionsgemisch mit 80 ml Wasser verdünnt und dreimal mit je 100 ml Petroläther extrahiert. Die organichen Phasen wurden zweimal mit je 100 ml Wasser gewaschen, über Magnesiumsulfat getrocknet, filtriert und eingeengt. Chromtographische Reinigung des Rohproduktes (2,1 g) an Kieselgel bei 0,5 bar mit Aethylacetat/Petroläther (Vol. 2:98) und Umkristallisation aus 20 ml Methanol ergab 1,26 g trans-4-[trans-4-(4-Pentenyl)cyclohexyl]cyclohexancarbonitril als farblose Kristalle mit Smp. (C-$S_B$) 20,1 °C, $S_B$-N 36,9 °C, Klp. (N-I) 54,8 °C.

In analoger Weise können folgende Verbindungen hergestellt werden:
trans-4-[trans-4-(4Z-Hexenyl)cyclohexyl]cyclohexancarbonitril;
trans-4-[trans-4-(4Z-Heptenyl)cyclohexyl]cyclohexancarbonitril;
trans-4-[trans-4-(4Z-Octenyl)cyclohexyl]cyclohexancarbonitril;
trans-4-[trans-4-(4Z-Nonenyl)cyclohexyl]cyclohexancarbonitril;
trans-4-[trans-4-(4Z-Decenyl)cyclohexyl]cyclohexancarbonitril.

Durch Umsetzung des in Stufe g) erhaltenen 3-[trans-4-(trans-4-Cyanocyclohexyl)cyclohexyl]-propionaldehyds in analoger Weise zu Stufe j) und gegebenenfalls E/Z-Isomerisierung in analoger Weise zu Beispiel 3k) können folgende Verbindungen hergestellt werden:
trans-4-[trans-4-(3-Butenyl)cyclohexyl]cyclohexancarbonitril; Smp. (C-N) 50,7 °C, Klp. (N-I) 82.7 °C;
trans-4-[trans-4-(3E-pentenyl)cyclohexyl]cyclohexancarbonitril; Smp. (C-N) 79,4 °C, Klp. (N-I) 99,5 °C;
trans-4-[trans-4-(3E-Hexenyl)cyclohexyl]cyclohexancarbonitril;
trans-4-[trans-4-(3E-Heptenyl)cyclohexyl]cyclohexancarbonitril;
trans-4-[trans-4-(3E-Octenyl)cyclohexyl]cyclohexancarbonitril;
trans-4-[trans-4-(3E-Nonenyl)cyclohexyl]cyclohexancarbonitril;
trans-4-[trans-4-(3E-Decenyl)cyclohexyl]cyclohexancarbonitril.

Durch Umsetzung des 4-(4-Oxocyclohexyl)benzamids in analoger Weise zu den Stufen h) und i), Acetalisierung des durch Kristallisation erhaltenen 4-(trans-4-Formylcyclohexyl)benzamids mit Aethylenglykol in Gegenwart von p-Toluolsulfonsäure, weitere Umsetzung des Dioxolans in analoger Weise zu den Stufen c) bis g), anschliessende Wittig-Reaktion in analoger Weise zu Stufe j) und gegebenenfalls E/Z-Isomerisierung in analoger Weise zu Beispiel 3k) können folgende Verbindungen hergestellt werden:
trans-4-(trans-4-Vinylcyclohexyl)cyclohexancarbonitril;
trans-4-[trans-4-(1E-Propenyl)cyclohexyl]cyclohexancarbonitril;
trans-4-[trans-4-(1E-Butenyl)cyclohexyl]cyclohexancarbonitril;
trans-4-[trans-4-(1E-Pentenyl)cyclohexyl]cyclohexancarbonitril;
trans-4-[trans-4-(1E-Hexenyl)cyclohexyl]cyclohexancarbonitril;
trans-4-[trans-4-(1E-Heptenyl)cyclohexyl]cyclohexancarbonitril;

trans-4-[trans-4-(1E-Octenyl)cyclohexyl]cyclohexancarbonitril;
trans-4-[trans-4-(1E-Nonenyl)cyclohexyl]cyclohexancarbonitril;
trans-4-[trans-4-(1E-Decenyl)cyclohexyl]cyclohexancarbonitril.

Ausgehend von 4-[2-(4-Oxocyclohexyl)äthyl]benzamid [herstellbar aus dem in Mol. Cryst. Liq. Cryst. 131, 327 (1985) beschriebenen Nitril] können in analoger Weise zur obigen Methode auch die folgenden Verbindungen hergestellt werden:

trans-4-[2-(trans-4-(4-Pentenyl)cyclohexyl)äthyl]cyclohexancarbonitril;
trans-4-[2-(trans-4-(4Z-Hexenyl)cyclohexyl)äthyl]cyclohexancarbonitril;
trans-4-[2-(trans-4-(4Z-Heptenyl)cyclohexyl)äthyl]cyclohexancarbonitril;
trans-4-[2-(trans-4-(4Z-Octenyl)cyclohexyl)äthyl]cyclohexancarbonitril;
trans-4-[2-(trans-4-(4Z-Nonenyl)cyclohexyl)äthyl]cyclohexancarbonitril;
trans-4-[2-(trans-4-(4Z-Decenyl)cyclohexyl)äthyl]cyclohexancarbonitril;
trans-4-[2-(trans-4-(3-Butenyl)cyclohexyl)äthyl]cyclohexancarbonitril;
trans-4-[2-(trans-4-(3E-Pentenyl)cyclohexyl)äthyl]cyclohexancarbonitril;
trans-4-[2-(trans-4-(3E-Hexenyl)cyclohexyl)äthyl]cyclohexancarbonitril;
trans-4-[2-(trans-4-(3E-Heptenyl)cyclohexyl)äthyl]cyclohexancarbonitril;
trans-4-[2-(trans-4-(3E-Octenyl)cyclohexyl)äthyl]cyclohexancarbonitril;
trans-4-[2-(trans-4-(3E-Nonenyl)cyclohexyl)äthyl]cyclohexancarbonitril;
trans-4-[2-(trans-4-(3E-Decenyl)cyclohexyl)äthyl]cyclohexancarbonitril;
trans-4-[2-(trans-4-Vinylcyclohexyl)äthyl]cyclohexancarbonitril;
trans-4-[2-(trans-4-(1E-Propenyl)cyclohexyl)äthyl]cyclohexancarbonitril;
trans-4-[2-(trans-4-(1E-Butenyl)cyclohexyl)äthyl]cyclohexancarbonitril;
trans-4-[2-(trans-4-(1E-Pentenyl)cyclohexyl)äthyl]cyclohexancarbonitril;
trans-4-[2-(trans-4-(1E-Hexenyl)cyclohexyl)äthyl]cyclohexancarbonitril;
trans-4-[2-(trans-4-(1E-Heptenyl)cyclohexyl)äthyl]cyclo hexancarbonitril;
trans-4-[2-(trans-4-(1E-Octenyl)cyclohexyl)äthyl]cyclohexancarbonitril;
trans-4-[2-(trans-4-(1E-Nonenyl)cyclohexyl)äthyl]cyclohexancarbonitril;
trans-4-[2-(trans-4-(1E-Decenyl)cyclohexyl)äthyl]cyclohexancarbonitril.

Beispiel 3

a) Eine Suspension von 109,6 g 4-(4-Nitrophenyl)cyclohexanon (herstellbar durch Nitrierung von 4-Phenylcyclohexanon) in 1 l Dioxan wurde mit 50 ml Triäthylamin und 2 g 5-prozentiger Palladium-Kohle versetzt und unter gutem Rühren bei Raumtemperatur und 0,3 bar Wasserstoffdruck hydriert. Nach 2 Stunden wurde das Gemisch filtriert. Das Filtrat wurde am Wasserstrahlvakuum bei einer Badtemperatur von 30 °C eingedampft und der Eindampfrückstand über Nacht im Trockenschrank am Wasserstrahlvakuum bei 40 °C getrocknet. Hierbei wurden 94,5 g 4-(4-Aminophenyl)cyclohexanon als weisse Kristalle mit Smp. 127-128 °C erhalten.

b) In einem Sulfierkolben wurden 200 ml 4N Schwefelsäure auf 80 °C erwärmt und dann mit ca. 5% einer Lösung von 37,9 g 4-(4-Aminophenyl)cyclohexanon in 200 ml 4N Schwefelsäure versetzt. Anschliessend wurden bei 80 °C innert 1,5 Stunden gleichzeitig die restliche Lösung von 4-(4-Aminophenyl)cyclohexanon sowie eine Lösung von 15,2 g Natriumnitrit in 45 ml Wasser zum Reaktionsgemisch zugetropft. Danach wurde das Gemisch bei 80 °C innert 30 Minuten tropfenweise mit einer Lösung von 9 g Natriumnitrit in 27 ml Wasser versetzt und noch 1 Stunde bei 80 °C weitergerührt. Nach dem Abkühlen des Reaktionsgemisches auf 0 °C wurden die ausgefallenen Kristalle abgenutscht, mit 200 ml kaltem Wasser gewaschen und im Trockenschrank am Wasserstrahlvakuum bei 60 °C bis zur Gewichtskonstanz getrocknet. Das kristalline Rohprodukt (34,6 g) wurde in 520 ml Aethylacetat suspendiert. Die Suspension wurde 1 Stunde zum Rückfluss erhitzt, dann mit 1,7 g Aktivkohle versetzt und dann noch 1 Stunde zum Rückfluss erhitzt. Anschliessend wurde das Gemisch genutscht (Nachwaschen mit 40 ml warmem Aethylacetat) und das Filtrat am Wasserstrahlvakuum bei einer Badtemperatur von 40 °C eingedampft. Trocknung des Eindampfrückstandes im Trockenschrank am Wasserstrahlvakuum bei 60 °C bis zur Gewichtskonstanz ergab 32,2 g 4-(4-Hydroxyphenyl)cyclohexanon als gelb-braune Kristalle mit Smp. 165-166 °C.

c) In einem Sulfierkolben wurde unter schwachem Stickstoffstrom eine Suspension von 3,8 g 4-(4-Hydroxyphenyl)cyclohexanon in 60 ml tert.Butylmethyläther mit 10,6 g 2-(1,3-Dioxolan-2-yl)äthyl-triphenylphosphoniumbromid und 2,2 g Kalium-tert.butylat versetzt. Das Gemisch wurde zunächst 30 Minuten gerührt, dann innert 2,25 Stunden bei 25 °C portionenweise mit weiteren 2,7 g Kalium-tert.butylat versetzt und noch 1 Stunde bei Raumtemperatur gerührt. Danach wurde das Reaktionsgemisch mit

weiteren 1,8 g 2-(1,3-Dioxolan-2-yl)äthyl-triphenylphosphoniumbromid und 0,45 g Kalium-tert.butylat versetzt und noch 1 Stunde gerührt. Anschliessend wurde das Reaktionsgemisch auf 80 ml Wasser gegossen und mit 11 ml 2N Schwefelsäure angesäuert. Die wässrige Phase wurde abgetrennt und zweimal mit je 80 ml tert.Butylmethyläther extrahiert. Die organischen Phasen wurden zweimal mit je 50 ml Wasser gewaschen, über Natriumsulfat getrocknet und genutscht. Das Filtrat wurde am Wasserstrahlvakuum bei einer Badtemperatur von 40 °C bis zur Gewichtskonstanz eingedampft. Das erhaltene rötliche Oel (10,9 g) wurde an Kieselgel mit Toluol und Toluol/tert.-Butylmethyläther (Vol. 20:1) chromatographisch getrennt. Eindampfen der Produktfraktionen am Wasserstrahlvakuum und Trocknung des Rückstandes im Trockenschrank am Wasserstrahlvakuum bei 60 °C bis zur Gewichtskonstanz ergab schliesslich 4,5 g 4-[4-[2-(1,3-Dioxolan-2-yl)äthyliden]cyclohexyl]phenol als gelbliche Kristalle mit Smp. 125,5-126,5 °C.

d) Eine Lösung von 36,5 g 4-[4-[2-(1,3-Dioxolan-2-yl)äthyliden]cyclohexyl]phenol in 500 ml Toluol und 50 ml Triäthylamin wurde in einem Hydrierautoklaven mit 3,2 g 5-prozentiger Platin-Kohle und 10 bar Wasserstoff bei 90 °C 1 Stunde hydriert. Anschliessend wurde das Gemisch genutscht und der Rückstand mit 30 ml warmem Toluol gewaschen. Das Filtrat wurde am Wasserstrahlvakuum bei einer Badtemperatur von 40 °C bis zur Gewichtskonstanz eingedampft. Die erhaltenen weissen Kristalle (37 g) wurden in 180 ml Methanol heiss gelöst. Die Lösung wurde auf Raumtemperatur abkühlen gelassen und dann 5 Stunden in den Kühlschrank gestellt. Anschliessend wurden die Kristalle abgenutscht, mit 50 ml kaltem Methanol gewaschen und im Trockenschrank am Wasserstrahlvakuum bei 60 °C getrocknet. Hierbei wurden 27,6 g 4-[trans-4-[2-(1,3-Dioxolan-2-yl)äthyl]cyclohexyl]phenol als weisse Kristalle mit Smp. 153,5-154,5 °C erhalten.

e) In einem Stahl-Rührautoklaven wurde ein Gemisch von 100 g 4-[trans 4-[2-(1,3-Dioxolan-2-yl)äthyl]-cyclohexyl]phenol, 10 g 5-prozentigem Rhodium/Aluminiumoxid und 1 l Aethylacetat mit 50 bar Wasserstoff bei 80 °C 90 Minuten hydriert. Dann wurde der Katalysator abgenutscht und mit 100 ml Aethylacetat gewaschen. Das Filtrat wurde eingedampft und der Rückstand bei 25 °C/0,4 mbar getrocknet. Hierbei wurden 101,75 g 4-[trans-4-[2-(1,3-Dioxolan-2-yl)äthyl]cyclohexyl]cyclohexanol enthaltend 59% cis-Isomer und 36% trans-Isomer erhalten.

f) Eine Lösung von 12,9 g Pyridiniumchlorochromat in 80 ml Methylenchlorid wurde bei Raumtemperatur unter Stickstoffbegasung innert 5 Minuten tropfenweise mit einer Lösung von 13,0 g 4-[trans-4-[2-(1,3-Dioxolan-2-yl)äthyl]cyclohexyl]cyclohexanol in 40 ml Methylenchlorid versetzt. Das Gemisch wurde noch 1 Stunde gerührt, dann mit 100 ml Diäthyläther verdünnt und filtriert und das Filtrat eingedampft. Der Eindampfrückstand wurde in 200 ml Diäthyläther aufgenommen, das Gemisch filtriert, das Filtrat eingedampft und dann dieses Vorgehen noch zweimal wiederholt. Danach wurde die erhaltenen, bräunliche feste Masse (12,1 g) an Kieselgel mit Aethylacetat/Petroläther (Vol. 1:4) chromatographisch getrennt. Kristallisation der farblosen Ketonfraktion aus 60 ml Aethylacetat und 200 ml Petroläther ergab schliesslich 7,0 g reines 4-[trans-4 -[2-(1,3-Dioxolan-2-yl)äthyl]cyclohexyl]cyclohexanon.

g) Eine Suspension von 2,97 g Natriumborhydrid in 300 ml Isopropanol wurde unter Stickstoffbegasung bei -70 °C tropfenweise mit einer Lösung von 11 g 4-[trans-4-[2-(1,3-Dioxolan-2-yl)äthyl]cyclohexyl]-cyclohexanon in 200 ml Isopropanol versetzt. Nach ca. 1 Stunde wurde das Reaktionsgemisch auf Raumtemperatur erwärmen gelassen, mit 500 ml 0,1N Salzsäure verdünnt und dreimal mit je 300 ml Methylenchlorid extrahiert. Die organischen Phasen wurden mit Wasser gewaschen, über Magnesiumsulfat getrocknet, filtriert und eingedampft. Kristallisation des Rückstandes (11 g) aus 500 ml Aethylacetat/Petroläther (Vol. 3:5) ergab 6,6 g reines trans-4-[trans-4 -[2-(1,3-Dioxolan-2-yl)äthyl]cyclohexyl]-cyclohexanol mit Smp. 129,5 °C.

h) In einem Rundkolben wurden unter Stickstoffbegasung 2,04 g Natriumhydrid als ca. 50-prozentige ölige Suspension vorgelegt und zweimal mit Pentan gewaschen. Dann wurden zum Natriumhydrid 40 ml trockenes Tetrahydrofuran und eine Lösung von 6,0 g trans 4-[trans-4 -[2-(1,3-Dioxolan-2-yl)äthyl]-cyclohexyl]cyclohexanol in 30 ml Tetrahydrofuran zugefügt. Das Gemisch wurde bei Raumtemperatur 30 Minuten gerührt, dann mit 4,0 ml Methyljodid versetzt und 2 Stunden zum Rückfluss erhitzt. Anschliessend wurde das Reaktionsgemisch abgekühlt, in 200 ml Diäthyläther aufgenommen und dreimal mit je 200 ml Wasser gewaschen. Die organische Phase wurde über Magnesiumsulfat getrocknet, filtriert und eingedampft, wobei 6,3 g 2-[2-[trans-4-(trans-4-Methoxycyclohexyl)cyclohexyl]äthyl] -1,3-dioxolan mit Smp. 74 °C erhalten wurden.

i) 6,2 g 2-[2-[trans-4-(trans-4-Methoxycyclohexyl)cyclohexyl]äthyl] -1,3-dioxolan wurden unter Stickstoffbegasung mit 100 ml Wasser, 50 ml Eisessig und 20 ml Dioxan versetzt. Das Gemisch wurde 1,5 Stunden bei 100 °C (Badtemperatur) gerührt, dann mit verdünnter Natriumhydrogencarbonat-Lösung neutralisiert und dreimal mit Diäthyläther extrahiert. Die vereinigten Aetherphasen wurden einmal mit Wasser und zweimal mit verdünnter Natriumhydrogencarbonat-Lösung gewaschen, über Magnesiumsulfat getrocknet, filtriert und eingedampft. Umkristallisation des erhaltenen rohen Aldehyds (4,34 g) aus 150 ml Petroläther bei -20 °C ergab 2,8 g 3-[trans-4-(trans -4-Methoxycyclohexyl)cyclohexyl]propionaldehyd in einer Reinheit von 96%.

j) 2,65 g Aethyltriphenylphosphoniumbromid wurden unter Argonbegasung in 40 ml tert.Butylmethyläther aufgeschlämmt. Die Suspension wurde bei Raumtemperatur mit 797 mg Kalium-tert.butylat versetzt und 1 Stunde gerührt. Anschliessend wurde das Gemisch auf 0 °C gekühlt, innert 3 Minuten tropfenweise mit einer Lösung von 1,1 g 3-[trans-4-(trans-4-Methoxycyclohexyl)cyclohexyl]propionaldehyd in 15 ml tert.Butylmethyläther versetzt und dann unter Rühren langsam auf Raumtemperatur erwärmen gelassen. Nach 2 Stunden wurde die hellgelbe Suspension in Diäthyläther/Wasser verteilt. Die wässrige Phase wurde abgetrennt und dreimal mit Diäthyläther gewaschen. Die organischen Phasen wurden zweimal mit Wasser gewaschen, über Magnesiumsulfat getrocknet, filtriert und eingedampft. Chromatographische Reinigung des gelben, festen Rohproduktes an Kieselgel mit Aethylacetat/Petroläther (Vol. 3:97) ergab 0,91 g trans-4-(3-Pentenyl)-1-(trans-4-methoxycyclohexyl)cyclohexan mit einem Z/E-Verhältnis von 86:11.

k) 0,91 g trans-4-(3-Pentenyl)-1-(trans-4-methoxycyclohexyl)cyclohexan (Z/E = 86:11) wurden unter Stickstoffbegasung mit 6 ml Toluol, 0,11 g Natriumbenzolsulfinat und 1 ml 1N Salzsäure versetzt. Das Gemisch wurde 15 Stunden bei 50 °C gerührt, dann auf 100 ml verdünnte Natriumhydrogencarbonat-Lösung gegossen und dreimal mit je 50 ml Diäthyläther extrahiert. Die vereinigten organischen Phasen wurden mit 100 ml verdünnter Natriumcarbonat-Lösung und mit 100 ml Wasser gewaschen, über Magnesiumsulfat getrocknet, filtriert und eingedampft. Das erhaltene gelbliche Oel (0,9 g) wurde an mit Silbernitrat imprägniertem Kieselgel mit Diäthyläther/Hexan (Vol. 1:9) chromatographisch gereinigt. Umkristallisation des erhaltenen Produktes (486 mg) aus 10 ml Methanol bei -20 °C ergab reines trans-4-(3E-Pentenyl)-1-(trans-4-methoxycyclohexyl)cyclohexan mit Smp. (C-N) 16,6 °C und Klp. (N-I) 43,7 °C.

In analoger Weise können folgende Verbindungen hergestellt werden:

trans-4-(3-Butenyl)-1-(trans-4-methoxycyclohexyl)cyclohexan, Smp. (C-N) -13,6 °C. Klp. (N-I) 18,0 °C;
trans-4-(3-Butenyl)-1-(trans-4-äthoxycyclohexyl)cyclohexan, Smp. (C-N) 13,1 °C, Klp. (N-I) 45,3 °C;
trans-4-(3-Butenyl)-1-(trans-4-propyloxycyclohexyl)cyclohexan;
trans-4-(3-Butenyl)-1-(trans-4-butyloxycyclohexyl)cyclohexan;
trans-4-(3-Butenyl)-1-(trans-4-pentyloxycyclohexyl)cyclohexan;
trans-4-(3-Butenyl)-1-(trans-4-hexyloxycyclohexyl)cyclohexan;
trans-4-(3E-Pentenyl)-1-(trans-4-äthoxycyclohexyl)cyclohexan, Smp. (C-N) 44,5 °C, Klp. (N-I) 76,5 °C;
trans-4-(3E-Pentenyl)-1-(trans-4-propyloxycyclohexyl)cyclohexan;
trans-4-(3E-Pentenyl)-1-(trans-4-butyloxycyclohexyl)cyclohexan;
trans-4-(3E-Pentenyl)-1-(trans-4-pentyloxycyclohexyl)cyclohexan;
trans-4-(3E-Pentenyl)-1-(trans-4-hexyloxycyclohexyl)cyclohexan;
trans-4-(3E-Hexenyl)-1-(trans-4-methoxycyclohexyl)cyclohexan;
trans-4-(3E-Hexenyl)-1-(trans-4-äthoxycyclohexyl)cyclohexan;
trans-4-(3E-Hexenyl)-1-(trans-4-propyloxycyclohexyl)cyclohexan;
trans-4-(3E-Hexenyl)-1-(trans-4-butyloxycyclohexyl)cyclohexan;
trans-4-(3E-Hexenyl)-1-(trans-4-pentyloxycyclohexyl)cyclohexan;
trans-4-(3E-Hexenyl)-1-(trans-4-hexyloxycyclohexyl)cyclohexan.

In analoger Weise zu den Stufen a) bis i) und weitere Umsetzung des erhaltenen Propionaldehyds in analoger Weise zu Beispiel 2, Stufen h), i) und j) können folgende Verbindungen hergestellt werden:

trans-4-(4-Pentenyl)-1-(trans-4-methoxycyclohexyl)cyclohexan, Smp. (C-$S_B$) 7,7 °C, Klp. ($S_B$-I) 14,0 °C;
trans-4-(4-Pentenyl)-1-(trans-4-äthoxycyclohexyl)cyclohexan, Smp. (C-$S_B$) 10,5 °C, Klp. ($S_B$-I) 43,1 °C;
trans-4-(4-Pentenyl)-1-(trans-4-propyloxycyclohexyl)cyclohexan;
trans-4-(4-Pentenyl)-1-(trans-4-butyloxycyclohexyl)cyclohexan;
trans-4-(4-Pentenyl)-1-(trans-4-pentyloxycyclohexyl)cyclohexan;
trans-4-(4-Pentenyl)-1-(trans-4-hexyloxycyclohexyl)cyclohexan;
trans-4-(4Z-Hexenyl)-1-(trans-4-methoxycyclohexyl)cyclohexan;
trans-4-(4Z-Hexenyl)-1-(trans-4-äthoxycyclohexyl)cyclohexan;
trans-4-(4Z-Hexenyl)-1-(trans-4-propyloxycyclohexyl)cyclohexan;
trans-4-(4Z-Hexenyl)-1-(trans-4-butyloxycyclohexyl)cyclohexan;
trans-4-(4Z-Hexenyl)-1-(trans-4-pentyloxycyclohexyl)cyclohexan;
trans-4-(4Z-Hexenyl)-1-(trans-4-hexyloxycyclohexyl)cyclohexan.

Durch Umsetzung des 4-(4-Hydroxyphenyl)cyclohexanons (Stufe b) in analoger Weise zu Beispiel 2, Stufen h) und i) Acetalisierung des erhaltenen 4-(trans-4-Formylcyclohexyl)phenols mit Aethylenglykol in Gegenwart von p-Toluolsulfonsäure und weitere Umsetzung des Dioxolans in analoger Weise zu den vorliegenden Stufen e) bis k) können folgende Verbindungen hergestellt werden:

trans-4-Vinyl-1-(trans-4-methoxycyclohexyl)cyclohexan;
trans-4-Vinyl-1-(trans-4-äthoxycyclohexyl)cyclohexan;
trans-4-Vinyl-1-(trans-4-propyloxycyclohexyl)cyclohexan;
trans-4-Vinyl-1-(trans-4-butyloxycyclohexyl)cyclohexan;
trans-4-Vinyl-1-(trans-4-pentyloxycyclohexyl)cyclohexan;
trans-4-Vinyl-1-(trans-4-hexyloxycyclohexyl)cyclohexan;
trans-4-(1E-Propenyl)-1-(trans-4-methoxycyclohexyl)cyclohexan;
trans-4-(1E-Propenyl)-1-(trans-4-äthoxycyclohexyl)cyclohexan;
trans-4-(1E-Propenyl)-1-(trans-4-propyloxycyclohexyl)cyclohexan;
trans-4-(1E-Propenyl)-1-(trans-4-butyloxycyclohexyl)cyclohexan;
trans-4-(1E-Propenyl)-1-(trans-4-pentyloxycyclohexyl)cyclohexan;
trans-4-(1E-Propenyl)-1-(trans-4-hexyloxycyclohexyl)cyclohexan;
trans-4-(1E-Butenyl)-1-(trans-4-methoxycyclohexyl)cyclohexan;
trans-4-(1E-Butenyl)-1-(trans-4-äthoxycyclohexyl)cyclohexan;
trans-4-(1E-Butenyl)-1-,trans-4-propyloxycyclohexyl)cyclohexan;
trans-4-(1E-Butenyl)-1-(trans-4-butyloxycyclohexyl)cyclohexan;
trans-4-(1E-Butenyl)-1-(trans-4-pentyloxycyclohexyl)cyclohexan;
trans-4-(1E-Pentenyl)-1-(trans-4-methoxycyclohexyl)cyclohexan;
trans-4-(1E-Pentenyl)-1-(trans-4-athoxycyclohexyl)cyclohexan;
trans-4-(1E-Pentenyl)-1-(trans-4-propyloxycyclohexyl)cyclohexan;
trans-4-(1E-Pentenyl)-1-(trans-4-butyloxycyclohexyl)cyclohexan.

Ausgehend von 4-(2-Phenyläthyl)cyclohexanon [herstellbar in analoger Weise zum in Mol. Cryst. Liq. Cryst. 131, 327 (1985) beschriebenen Nitril] können nach der obigen Methode auch die folgenden Verbindungen hergestellt werden:

4-[2-[trans-4-(2-(1,3-Dioxolan-2-yl)äthyl)cyclohexyl]äthyl]cyclohexanon;
trans-4-(3-Butenyl)-1-[2-(trans-4-methoxycyclohexyl)äthyl]cyclohexan;
trans-4-(3-Butenyl)-1-[2-(trans-4-äthoxycyclohexyl)äthyl]cyclohexan;
trans-4-(3-Butenyl)-1-[2-(trans-4-propyloxycyclohexyl)äthyl]cyclohexan;
trans-4-(3-Butenyl)-1-[2-(trans-4-butyloxycyclohexyl)äthyl]cyclohexan;
trans-4-(3-Butenyl)-1-[2-(trans-4-pentyloxycyclohexyl)äthyl]cyclohexan;
trans-4-(3-Butenyl)-1-[2-(trans-4-hexyloxycyclohexyl)äthyl]cyclohexan;
trans-4-(3E-Pentenyl)-1-[2-(trans-4-methoxycyclohexyl)äthyl]cyclohexan;
trans-4-(3E-Pentenyl)-1-[2-(trans-4-äthoxycyclohexyl)äthyl]cyclohexan;
trans-4-(3E-Pentenyl)-1-[2-(trans-4-propyloxycyclohexyl)äthyl]cyclohexan;
trans-4-(3E-Pentenyl)-1-[2-(trans-4-butyloxycyclohexyl)äthyl]cyclohexan;
trans-4-(3E-Pentenyl)-1-[2-(trans-4-pentyloxycyclohexyl)äthyl]cyclohexan;
trans-4-(3E-Pentenyl)-1-[2-(trans-4-hexyloxycyclohexyl)äthyl]cyclohexan;
trans-4-(3E-Hexenyl)-1-[2-(trans-4-methoxycyclohexyl)äthyl]cyclohexan;
trans-4-(3E-Hexenyl)-1-[2-(trans-4-äthoxycyclohexyl)äthyl]cyclohexan;
trans-4-(3E-Hexenyl)-1-[2-(trans-4-propyloxycyclohexyl)äthyl]cyclohexan;
trans-4-(3E-Hexenyl)-1-[2-(trans-4-butyloxycyclohexyl)äthyl]cyclohexan;
trans-4-(3E-Hexenyl)-1-[2-(trans-4-pentyloxycyclohexyl)äthyl]cyclohexan;
trans-4-(3E-Hexenyl)-1-[2-(trans-4-hexyloxycyclohexyl)äthyl]cyclohexan;
trans-4-(4-Pentenyl)-1-[2-(trans-4-methoxycyclohexyl)äthyl]cyclohexan;
trans-4-(4-Pentenyl)-1-[2-(trans-4-äthoxycyclohexyl)äthyl]cyclohexan;
trans-4-(4-Pentenyl)-1-[2-(trans-4-propyloxycyclohexyl)äthyl]cyclohexan;
trans-4-(4-Pentenyl)-1-[2-(trans-4-butyloxycyclohexyl)äthyl]cyclohexan;
trans-4-(4-Pentenyl)-1-[2-(trans-4-pentyloxycyclohexyl)äthyl]cyclohexan;
trans-4-(4-Pentenyl)-1-[2-(trans-4-hexyloxycyclohexyl)äthyl]cyclohexan;
trans-4-(4Z-Hexenyl)-1-[2-(trans-4-methoxycyclohexyl)äthyl]cyclohexan;
trans-4-(4Z-Hexenyl)-1-[2-(trans-4-äthoxycyclohexyl)äthyl]cyclohexan;
trans-4-(4Z-Hexenyl)-1-[2-(trans-4-propyloxycyclohexyl)äthyl]cyclohexan;
trans-4-(4Z-Hexenyl)-1-[2-(trans-4-butyloxycyclohexyl)äthyl]cyclohexan;
trans-4-Vinyl-1-[2-(trans-4-methoxycyclohexyl)äthyl]cyclohexan;
trans-4-Vinyl-1-[2-(trans-4-äthoxycyclohexyl)äthyl]cyclohexan;
trans-4-Vinyl-1-[2-(trans-4-propyloxycyclohexyl)äthyl]cyclohexan;
trans-4-Vinyl-1-[2-(trans-4-butyloxycyclohexyl)äthyl]cyclohexan;
trans-4-Vinyl-1-[2-(trans-4-pentyloxycyclohexyl)äthyl]cyclohexan;

trans-4-Vinyl-1-[2-(trans-4-hexyloxycyclohexyl)äthyl]cyclohexan;
trans-4-(1E-Propenyl)-1-[2-(trans-4-methoxycyclohexyl)äthyl]cyclohexan;
trans-4-(1E-Propenyl)-1-[2-(trans-4-äthoxycyclohexyl)äthyl]cyclohexan;
trans-4-(1E-Propenyl)-1-[2-(trans-4-propyloxycyclohexyl)äthyl]cyclohexan;
trans-4-(1E-Propenyl)-1-[2-(trans-4-butyloxycyclohexyl)äthyl]cyclohexan;
trans-4-(1E-Propenyl)-1-[2-(trans-4-pentyloxycyclohexyl)äthyl]cyclohexan;
trans-4-(1E-Propenyl)-1-[2-(trans-4-hexyloxycyclohexyl)äthyl]cyclohexan;
trans-4-(1E-Butenyl)-1-[2-(trans-4-methoxycyclohexyl)äthyl]cyclohexan;
trans-4-(1E-Butenyl)-1-[2-(trans-4-äthoxycyclohexyl)äthyl]cyclohexan;
trans-4-(1E-Butenyl)-1-[2-(trans-4-propyloxycyclohexyl)äthyl]cyclohexan;
trans-4-(1E-Butenyl)-1-[2-(trans-4-butyloxycyclohexyl)äthyl]cyclohexan;
trans-4-(1E-Butenyl)-1-[2-(trans-4-pentyloxycyclohexyl)äthyl]cyclohexan;
trans-4-(1E-Pentenyl)-1-[2-(trans-4-methoxycyclohexyl)äthyl]cyclohexan;
trans-4-(1E-Pentenyl)-1-[2-(trans-4-äthoxycyclohexyl)äthyl]cyclohexan;
trans-4-(1E-Pentenyl)-1-[2-(trans-4 propyloxycyclohexyl)athyl]cyclohexan;
trans-4-(1E-Pentenyl)-1-[2-(trans-4-butyloxycyclohexyl)äthyl]cyclohexan.

Beispiel 4

a) Eine Grignard-Lösung aus 3,59 g Magnesium und 16,75 ml 1-Brom-4-fluorbenzol in 70 ml Tetrahydrofuran wurde bei 0 °C innert 30 Minuten tropfenweise mit einer Lösung von 33,1 g 4-[trans-4 -[2-(1,3-Dioxolan-2-yl)äthyl]cyclohexyl]cyclohexanon (hergestellt nach Beispiel 3) in 90 ml Tetrahydrofuran versetzt. Das Reaktionsgemisch wurde noch 4 Stunden bei Raumtemperatur gerührt und dann 1,5 Stunden zum Sieden erhitzt. Anschliessend wurde das Reaktionsgemisch abkühlen gelassen, mit 100 ml Diäthyläther verdünnt und mit 80 ml halbgesättigter Ammoniumchlorid-Lösung gewaschen. Die wässrige Phase wurde mit 100 ml Diäthyläther nachextrahiert. Die vereinigten organischen Phasen wurden dreimal mit je 60 ml gesättigter Kochsalzlösung gewaschen, über Natriumsulfat getrocknet, filtriert und eingedampft. Hierbei wurden 41,0 g rohes 1-[4-[trans-4-(2-(1,3-Dioxolan-2-yl)äthyl)cyclohexyl] -1-hydroxycyclohexyl]-4-fluorbenzol erhalten.
b) Eine Lösung von 41,0 g rohem 1-[4-[trans-4-(2-(1,3-Dioxolan-2-yl)äthyl]cyclohexyl] -1-hydroxycyclohexyl]-4-fluorbenzol in 225 ml m-Xylol wurde mit 15 g Kaliumhydrogensulfat versetzt und das Gemisch unter Rühren 11 Stunden zum Sieden erhitzt. Nach dem Erkalten wurde das Salz abfiltriert. Das Filtrat wurde mit 250 ml Diäthyläther verdünnt, mit 200 ml gesättigter Natriumhydrogencarbonat-Lösung und zweimal mit je 150 ml Wasser gewaschen, über Natriumsulfat getrocknet und eingeengt. Hierbei wurden 30,3 g rohes 1-[4-[trans-4-(2-(1,3-Dioxolan-2-yl)äthyl)cyclohexyl] -1-cyclohexenyl]-4-fluorbenzol erhalten.
c) Eine Lösung von 31,5 g rohem 1-[4-[trans-4-(2-(1,3-Dioxolan-2-yl)äthyl)cyclohexyl] -1-cyclohexenyl]-4-fluorbenzol und 0,5 ml Triäthylamin in 1 l Toluol wurde mit 4,5 g 5-prozentiger Palladium-Kohle bei Raumtemperatur und Normaldruck hydriert, bis die Wasserstoffaufnahme zum Stillstand kam. Der Katalysator wurde abfiltriert und das Filtrat eingedampft. Das erhaltene 1-[4-[trans-4-(2-(1,3-Dioxolan -2-yl)äthyl)cyclohexyl]cyclohexyl] 4-fluorbenzol (31,1 g; cis/trans-Verhältnis ca. 1:1) wurde zur Isomerisierung mit einer Lösung von 10,0 g Kalium-tert.-butylat in 310 ml N,N-Dimethylformamid versetzt und 23 Stunden auf 105 °C erhitzt. Anschliessend wurde das Reaktionsgemisch auf 400 g Eis und 100 ml gesättigte Natriumhydrogencarbonat-Lösung gegossen. Das Gemisch wurde einmal mit 500 ml und zweimal mit je 250 ml Diäthyläther extrahiert. Die organischen Phasen wurden dreimal mit je 200 ml Wasser gewaschen, über Natriumsulfat getrocknet, filtriert und eingedampft. Hierbei wurden 30,5 g überwiegend festes 1-[trans-4-[trans-4-(2-(1,3-Dioxolan -2-yl)äthyl)cyclohexyl]cyclohexyl]-4-fluorbenzol erhalten, welches ohne zusätzliche Reinigung weiterverwendet wurde.
In analoger Weise können folgende Verbindungen hergestellt werden:
1-[trans-4-[trans-4-(2-(1,3-Dioxolan-2-yl)äthyl)cyclohexyl]cyclohexyl]-3,4-difluorbenzol;
1-[trans-4-[trans-4-(2-(1,3-Dioxolan-2-yl)äthyl)cyclohexyl]cyclohexyl]-3-chlor-4-fluorbenzol;
1-[trans-4-[trans-4-(2-(1,3-Dioxolan-2-yl)äthyl)cyclohexyl]cyclohexyl]-3-fluor-4-chlorbenzol;
1-[trans-4-[trans-4-(2-(1,3-Dioxolan-2-yl)äthyl)cyclohexyl]cyclohexyl]-4-chlorbenzol;
1-[trans-4-[2-[trans-4-(2-(1,3-Dioxolan-2-yl)äthyl)cyclohexyl]äthyl]cyclohexyl]-4-fluorbenzol;
1-[trans-4-[2-[trans-4-(2-(1,3-Dioxolan-2-yl)äthyl)cyclohexyl]äthyl]cyclohexyl]-3,4-difluorbenzol;
1-[trans-4-[2-[trans-4-(2-(1,3-Dioxolan-2-yl)äthyl)cyclohexyl]äthyl]cyclohexyl]-3-chlor-4-fluorbenzol;
1-[trans-4-[2-[trans-4-(2-(1,3-Dioxolan-2-yl)äthyl)cyclohexyl]äthyl]cyclohexyl]-4-chlorbenzol]:
1-[trans-4-[2-[trans-4-(2-(1,3-Dioxolan-2-yl)äthyl)cyclohexyl]äthyl]cyclohexyl]-3-fluor-4-chlorbenzol.

Beispiel 5

a) Eine Lösung von 3,1 g trans-4-[trans-4 -[2-(1,3-Dioxolan-2-yl)äthyl]cyclohexyl]cyclohexancarbonsäure (hergestellt nach Beispiel 2) in 50 ml trockenem Diäthyläther wird bei 0 °C mit 380 mg Lithiumaluminiumhydrid versetzt und dann 4 Stunden zum Rückfluss erhitzt. Danach wird das Reaktionsgemisch abgekühlt, mit Eiswasser und Ammoniumchlorid-Lösung versetzt und mit Diäthyläther extrahiert. Die organische Phase wird mit Wasser gewaschen, über Magnesiumsulfat getrocknet, filtriert und eingedampft. Das erhaltene Rohprodukt von [trans-4-[trans-4 -(2-(1,3-Dioxolan-2-yl)äthyl)cyclohexyl]-cyclohexyl]methanol wird ohne zusätzliche Reinigung weiterverwendet.
b) Eine Lösung von 2,96 g rohem [trans-4-[trans-4-(2-(1,3-Dioxolan-2-yl)äthyl)cyclohexyl]cyclohexyl]-methanol in 20 ml trockenem Pyridin wird bei 0 °C mit 2,1 g p-Toluolsulfonylchlorid versetzt. Das Gemisch wird 15 Stunden bei Raumtemperatur gerührt, dann mit 200 ml Methylenchlorid verdünnt und mehrmals mit Wasser gewaschen. Die organische Phase wird über Magnesiumsulfat getrocknet, filtriert und eingedampft. Chromatographische Reinigung des erhaltenen Rohproduktes an Kieselgel mit Aethylacetat/Petroläther (Vol. 1:9) ergibt [trans-4-[trans-4 -(2-(1,3-Dioxolan-2-yl)äthyl)cyclohexyl]cyclohexyl]-methyl -p-toluolsulfonat.
c) Eine Lösung von 0,33 g Kaliumhydroxid in 7 ml 95-prozentigem Aethanol wird mit 1,12 g p-Fluorphenol versetzt. Danach wird das Gemisch mit einer Lösung von 2,26 g [trans-4-[trans-4 -(2-(1,3-Dioxolan-2-yl)äthyl)cyclohexyl]cyclohexyl]methyl -p-toluolsulfonat in 23 ml Aethanol versetzt und 24 Stunden bei 80 °C (Badtemperatur) gerührt. Anschliessend wird das Reaktionsgemisch in 1N Salzsäure und Methylenchlorid verteilt. Die organische Phase wird mehrmals mit Wasser gewaschen, über Magnesiumsulfat getrocknet, filtriert und eingedampft. Chromatographische Reinigung des erhaltenen Rohproduktes an Kieselgel mit Aethylacetat/Petroläther ergibt 1-[[trans-4-(trans-4- (2-(1,3-Dioxolan-2-yl)-äthyl)cyclohexyl)cyclohexyl]methoxy] -4-fluorbenzol.

In analoger Weise können folgende Verbindungen hergestellt werden:
1-[[trans-4-(trans-4-(2-(1,3-Dioxolan-2-yl)-äthyl)cyclohexyl)cyclohexyl]methoxy]-3,4-difluorbenzol;
1-[[trans-4-(trans-4-(2-(1,3-Dioxolan-2-yl)-äthyl)cyclohexyl)cyclohexyl]methoxy]-3-chlor-4-fluorbenzol;
1-[[trans-4-(trans-4-(2-(1,3-Dioxolan-2-yl)-äthyl)cyclohexyl)cyclohexyl]methoxy]-4-chlorbenzol;
1-[[trans 4-(trans 4-(2-(1,3-Dioxolan-2-yl)-äthyl)cyclohexyl)cyclohexyl]methoxy]-3-fluor-4-chlorbenzol.

Beispiel 6

a) Eine Lösung von 3,1 g Pyridiniumchlorochromat in 20 ml Methylenchlorid wird bei Raumtemperatur tropfenweise mit einer Lösung von 3 g [trans-4-[trans-4 -(2-(1,3-Dioxolan-2-yl)äthyl)cyclohexyl]-cyclohexyl]methanol (herstellbar nach Beispiel 5) in 10 ml Methylenchlorid versetzt. Das Gemisch wird noch 1 Stunde gerührt, dann mit 50 ml Diäthyläther verdünnt und filtriert. Das Filtrat wird eingedampft, der Eindampfrückstand in 50 ml Diäthyläther aufgenommen und die erhaltene Lösung nochmals filtriert. Chromatographische Reinigung an Kieselgel mit Aethylacetat/Petroläther (Vol. 1:4) und Kristallisation aus Aethylacetat/Hexan ergibt schliesslich trans-4-[trans-4-[2-(1,3-Dioxolan -2-yl)äthyl]cyclohexyl]-cyclohexancarboxaldehyd.
b) 3 g p-Fluorbenzyl-triphenylphosphoniumbromid (herstellbar aus p-Fluorbenzylbromid und Triphenylphosphin) werden in 50 ml tert.Butylmethyläther aufgeschlämmt. Die Suspension wird bei Raumtemperatur mit 0,75 g Kalium-tert.butylat versetzt und 1,5 Stunden gerührt. Anschliessend wird das Gemisch bei 0 °C innert 5 Minuten tropfenweise mit einer Lösung von 1,40 g trans-4-[trans-4-[2-(1,3-Dioxoalan-2-yl)-athyl]cyclohexyl]cyclohexancarboxaldehyd in 25 ml tert.Butylmethyläther versetzt und noch 24 Stunden bei Raumtemperatur gerührt. Danach wird das Reaktionsgemisch in Diäthyläther aufgenommen, mehrmals mit Wasser gewaschen, über Magnesiumsulfat getrocknet, filtriert und eingeengt. Chromatographische Reinigung des erhaltenen Rohproduktes an Kieselgel mit Aethylacetat/Petroläther (Vol. 3:97) ergibt β-[trans-4-[trans-4-(2-(1,3-Dioxolan -2-yl)äthyl)cyclohexyl]cyclohexyl]-4-fluorstyrol.
c) Eine Lösung von 1 g β-[trans-4-[trans-4-(2-(1,3-Dioxolan-2-yl)äthyl)cyclohexyl]cyclohexyl]-4-fluorstyrol in 10 ml Toluol und 5 ml Aethanol wird mit 500 mg 5-prozentiger Palladium-Kohle bei Raumtemperatur und Normaldruck bis zum Stillstand der Wasserstoffaufnahme hydriert. Anschliessend wird die schwarze Suspension filtriert. Eindampfen des Filtrates ergibt 1-[2-[trans-4-[trans-4-(2-(1,3-Dioxolan -2-yl)äthyl)-cyclohexyl]cyclohexyl]äthyl]-4-fluorbenzol.

In analoger Weise können folgende Verbindungen hergestellt werden:
1-[2-[trans-4-[trans-4-(2-(1,3-Dioxolan-2-yl)äthyl)cyclohexyl]cyclohexyl]äthyl]-3,4-difluorbenzol;
1-2-[trans-4-[trans-4-(2-(1,3-Dioxolan-2-yl)äthyl)cyclohexyl]cyclohexyl]äthyl]-3-chlor-4-fluorbenzol;
1-[2-[trans-4-[trans-4-(2-(1,3-Dioxolan-2-yl)äthyl)cyclohexyl]cyclohexyl]äthyl]-4-chlorbenzol;

1-[2-[trans-4-[trans-4-(2-(1,3-Dioxolan-2-yl)äthyl)cyclohexyl]cyclohexyl]äthyl]-3-fluor-4-chlorbenzol.

Beispiel 7

Eine Lösung von 1,7 g 4-Fluorbenzoylchlorid in 5 ml Pyridin wird mit 2,8 g trans-4-[trans -4-[2-(1,3-Dioxolan-2-yl)äthyl]cyclohexyl]cyclohexanol (hergestellt nach Beispiel 3) versetzt und 12 Stunden bei Raumtemperatur gerührt. Danach wird das Reaktionsgemisch auf Eiswasser gegossen und dreimal mit Diäthyläther extrahiert. Die organischen Phasen werden nacheinander mit gesättigter Natriumhydrogencarbonat-Lösung, mit 10-prozentiger Salz säure, mit gesättigter Natriumhydrogencarbonat-Lösung und mit Wasser gewaschen, dann über Magnesiumsulfat getrocknet, filtriert und eingeengt. Chromatographische Reinigung des erhaltenen Rohproduktes an Kieselgel mit Aethylacetat/Petroläther (Vol. 3:97) ergibt 4-Fluorbenzoesäure-trans-4-[trans-4-[2-(1,3-dioxolan -2-yl)äthyl]cyclohexyl]cyclohexylester.

In analoger Weise können folgende Verbindungen hergestellt werden:

3,4-Difluorbenzoesäure-trans-4-[trans-4-[2-(1,3-dioxolan-2-yl)äthyl]cyclohexyl]cyclohexylester;
3-Chlor-4-fluorbenzoesäure-trans-4-[trans-4-[2-(1,3-dioxolan-2-yl)äthyl]cyclohexyl]cyclohexylester;
4-Chlorbenzoesäure-trans-4-[trans-4-[2-(1,3-dioxolan-2-yl)äthyl]cyclohexyl]cyclohexylester;
3-Fluor-4-chlorbenzoesäure-trans-4-[trans-4-[2-(1,3-dioxolan-2-yl)äthyl]cyclohexyl]cyclohexylester.

Beispiel 8

2,04 g Natriumhydrid als ca. 50-prozentige ölige Suspension werden unter Stickstoffbegasung vorgelegt und zweimal mit Pentan gewaschen. Dann werden zum Natriumhydrid 40 ml trockenes Tetrahydrofuran und eine Lösung von 6,0 g trans-4-[trans -4-[2-(1,3-Dioxolan-2-yl)äthyl]cyclohexyl]cyclohexanol (hergestellt nach Beispiel 2) in 30 ml Tetrahydrofuran zugefügt. Das Gemisch wird bei Raumtemperatur 30 Minuten gerührt, dann mit 6,03 g 4-Fluorbenzylbromid versetzt und 2 Stunden zum Rückfluss erhitzt. Anschliessend wird das Reaktionsgemisch abgekühlt, in 200 ml Diäthyläther aufgenommen und dreimal mit je 200 ml Wasser gewaschen. Die organische Phase wird über Magnesiumsulfat getrocknet, filtriert und eingedampft wobei trans-4-[trans-4-[2-(1,3-Dioxolan -2-yl)äthyl]cyclohexyl]cyclohexyl-4-fluorbenzyläther erhalten wird.

In analoger Weise können folgende Verbindungen hergestellt werden:

trans-4-[trans-4-[2-(1,3-Dioxolan-2-yl)äthyl]cyclohexyl]cyclohexyl-3,4-difluorbenzyläther;
trans-4-[trans-4-[2-(1,3-Dioxolan-2-yl)äthyl]cyclohexyl]cyclohexyl-3-chlor-4-fluorbenzyläther;
trans-4-[trans-4-[2-(1,3-Dioxolan-2-yl)äthyl]cyclohexyl]cyclohexyl-4-chlorbenzyläther;
trans-4-[trans-4-[2-(1,3-Dioxolan-2-yl)äthyl]cyclohexyl]cyclohexyl-3-fluor-4-chlorbenzyläther.

Beispiel 9

a) Ein Gemisch von 29,1 g rohem 1-[trans-4-[trans-4-(2-(1,3-Dioxolan -2-yl)äthyl)cyclohexyl]cyclohexyl]-4-fluorbenzol, 200 ml Dioxan, 200 ml Eisessig und 400 ml Wasser wurde unter Rühren und Stickstoffbegasung 5 Stunden zu leichtem Sieden erhitzt (Badtemperatur 115 °C). Dann wurde das Reaktionsgemisch auf 500 g Eis gegossen. Die wässrige Phase wurde abgetrennt und dreimal mit je 400 ml Diathyläther extrahiert. Die vereinigten organischen Phasen wurden mit 500 ml gesättigter Natriumhydrogencarbonat-Lösung und mit 500 ml Wasser gewaschen, über Natriumsulfat getrocknet, filtriert und eingeengt. Hierbei wurden 25,2 g roher, fester 3-[trans-4-[trans -4-(4-Fluorphenyl)cyclohexyl]cyclohexyl]-propionaldehyd erhalten.

b) Eine Suspension von 9,1 g Methoxymethyl-triphenylphosphoniumchlorid in 50 ml Diäthyläther wurde unter Stickstoffbegasung mit 2,85 g Kalium-tert.butylat versetzt. Die rote Suspension wurde noch 30 Minuten bei Raumtemperatur gerührt und dann bei 0 °C tropfenweise mit einer Lösung von 5,43 g rohem 3-[trans-4 -[trans-4-(4-Fluorphenyl)cyclohexyl]cyclohexyl]propionaldehyd in 30 ml trockenem Diäthyläther versetzt. Das Reaktionsgemisch wurde noch 90 Minuten bei Raumtemperatur gerührt, dann auf 300 ml Hexan gegossen und filtriert. Chromatogrpahische Reinigung des eingeengten Filtrates an Kieselgel mit Hexan ergab 4,9 g festes 1-[trans-4-[trans-4-(4-Methoxy -3-butenyl)cyclohexyl]cyclohexyl]-4-fluorbenzol.

c) Unter Rühren und Stickstoffbegasung wurde ein Gemisch von 2,48 g 1-[trans-4-[trans-4-(4-Methoxy -3-butenyl)cyclohexyl]cyclohexyl]-4-fluorbenzol, 30 ml Dioxan, 20 ml Eisessig und 40 ml Wasser 6 Stunden zu leichtem Sieden erhitzt (Badtemperatur 115 °C). Nach dem Erkalten wurde die Suspension mit 70 ml Wasser verdünnt. Die wässrige Phase wurde abgetrennt und dreimal mit je 80 ml Diäthyläther extrahiert. Die vereinigten organischen Phasen wurden zweimal mit je 100 ml Wasser gewaschen, über Natriumsulfat getrocknet, filtriert und eingeengt. Hierbei wurden 2,3 g fester 4-[trans-4-[trans -4-(4-Fluorphenyl)cyclohexyl]cyclohexyl]butyraldehyd erhalten.

d) Eine Suspension von 5,19 g Methyl-triphenylphosphoniumbromid in 80 ml Diäthyläther wurde unter Stickstoffbegasung mit 1,55 g Kalium-tert.butylat versetzt. Die gelbe Suspension wurde noch 45 Minuten bei Raumtemperatur gerührt und dann bei 0 °C tropfenweise mit einer Lösung von 2,3 g 4-[trans-4-[trans -4-(4-Fluorphenyl)cyclohexyl]cyclohexyl]butyraldehyd versetzt. Das Reaktionsgemisch wurde noch 2 Stunden bei 0 °C gerührt und dann mit 60 ml Wasser verdünnt. Die wässrige Phase wurde abgetrennt und zweimal mit je 60 ml Hexan extrahiert. Die vereinigten organischen Phasen wurden mit Wasser neutral gewaschen, über Natriumsulfat getrocknet, filtriert und eingeengt. Chromatographische Reinigung des Rückstandes an Kieselgel mit Hexan ergab 1,89 g Rohprodukt. Nach zweimaliger Umkristallisation aus Aceton bei -20 °C wurden 1,22 g 1-[trans-4-[trans-4-(4-Pentenyl)cyclohexyl]cyclohexyl] -4-fluorbenzol mit Smp. (C-N) 65,7 °C und Klp. (N-I) 129,7 °C erhalten.

In analoger Weise können die in Beispiel 1 genannten 4-Alkenylverbindungen sowie die folgenden Verbindungen hergestellt werden:

1-[trans-4-[trans-4-(4-Pentenyl)cyclohexyl]cyclohexyl-3,4-difluorbenzol;
1-[trans-4-[trans-4-(4-Pentenyl)cyclohexyl]cyclohexyl-3-chlor-4-fluorbenzol;
1-[trans-4-[trans-4-(4-Pentenyl)cyclohexyl]cyclohexyl-4-chlorbenzol;
1-[trans-4-[trans-4-(4-Pentenyl)cyclohexyl]cyclohexyl-3-fluor-4-chlorbenzol;
1-[trans-4-[2-(trans-4-(4-Pentenyl)cyclohexyl)äthyl]cyclohexyl]-4-fluorbenzol;
1-[trans-4-[2-(trans-4-(4-Pentenyl)cyclohexyl)äthyl]cyclohexyl]-3,4-difluorbenzol;
1-[trans-4-[2-(trans-4-(4-Pentenyl)cyclohexyl)äthyl]cyclohexyl]-3-chlor-4-fluorbenzol;
1-[trans-4-[2-(trans-4-(4-Pentenyl)cyclohexyl)äthyl]cyclohexyl]-4-chlorbenzol;
1-[trans-4-[2-(trans-4-(4-Pentenyl)cyclohexyl)äthyl]cyclohexyl]-3-fluor-4-chlorbenzol;
1-[[trans-4-(trans-4-(4-Pentenyl)cyclohexyl)cyclohexyl]methoxy]-4-fluorbenzol;
1-[[trans-4-(trans-4-(4-Pentenyl)cyclohexyl)cyclohexyl]methoxy]-3,4-difluorbenzol;
1-[[trans-4-(trans-4-(4-Pentenyl)cyclohexyl)cyclohexyl]methoxy]-3-chlor-4-fluorbenzol;
1-[[trans-4-(trans-4-(4-Pentenyl)cyclohexyl)cyclohexyl]methoxy]-4-chlorbenzol;
1-[[trans-4-(trans-4-(4-Pentenyl)cyclohexyl)cyclohexyl]methoxy]-3-fluor-4-chlorbenzol;
1-[2-[trans-4-(trans-4-(4-Pentenyl)cyclohexyl)cyclohexyl]äthyl]-4-fluorbenzol, Smp. (C-$S_B$) 7,1 °C, Phasenübergang ($S_B$-N) 93 °C, Klp. (N-I) 116,6 °C;
1-[2-[trans-4-(trans-4-(4-Pentenyl)cyclohexyl)cyclohexyl]äthyl]-3,4-difluorbenzol;
1-[2-[trans-4-(trans-4-(4-Pentenyl)cyclohexyl)cyclohexyl]äthyl]-3-chlor-4-fluorbenzol;
1-[2-[trans-4-(trans-4-(4-Pentenyl)cyclohexyl)cyclohexyl]äthyl]-4-chlorbenzol;
1-[2-[trans-4-(trans-4-(4-Pentenyl)cyclohexyl)cyclohexyl]äthyl]-3-fluor-4-chlorbenzol;
4-Fluorbenzoesäure-trans-4-[trans-4-(4-pentenyl)cyclohexyl]cyclohexylester;
3,4-Difluorbenzoesäure-trans-4-[trans-4-(4-pentenyl)cyclohexyl]cyclohexylester;
3-Chlor-4-fluorbenzoesäure-trans-4-[trans-4-(4-pentenyl)cyclohexyl]cyclohexylester;
4-Chlorbenzoesäure-trans-4-[trans-4-(4-pentenyl)cyclohexyl]cyclohexylester;
3-Fluor-4-chlorbenzoesäure-trans-4-[trans-4-(4-pentenyl)cyclohexyl]cyclohexylester;
trans-4-[trans-4-(4-Pentenyl)cyclohexyl]cyclohexyl-4-fluorbenzyläther;
trans-4-[trans-4-(4-Pentenyl)cyclohexyl]cyclohexyl-3,4-difluorbenzyläther;
trans-4-[trans-4-(4-Pentenyl)cyclohexyl]cyclohexyl-3-chlor-4-fluorbenzyläther;
trans-4-[trans-4-(4-Pentenyl)cyclohexyl]cyclohexyl-4-chlorbenzyläther;
trans-4-[trans-4-(4-Pentenyl)cyclohexyl]cyclohexyl-3-fluor-4-chlorbenzyläther;
1-[trans-4-[trans-4-(4Z-Hexenyl)cyclohexyl]cyclohexyl]-4-fluorbenzol;
1-[trans-4-[trans-4-(4Z-Hexenyl)cyclohexyl]cyclohexyl]-3,4-difluorbenzol;
1-[trans-4-[2-(trans-4-(4Z-Hexenyl)cyclohexyl)äthyl]cyclohexyl]-4-fluorbenzol;
1-[trans-4-[2-(trans-4-(4Z-Hexenyl)cyclohexyl)äthyl]cyclohexyl]-3,4-difluorbenzol;
1-[[trans-4-(trans-4-(4Z-Hexenyl)cyclohexyl)cyclohexyl]methoxy]-4-fluorbenzol;
1-[[trans-4-(trans-4-(4Z-Hexenyl)cyclohexyl)cyclohexyl]methoxy]-3,4-difluorbenzol;
1-[2-[trans-4-(trans-4-(4Z-Hexenyl)cyclohexyl)cyclohexyl]äthyl]-4-fluorbenzol;
1-[2-[trans-4-(trans-4-(4Z-Hexenyl)cyclohexyl)cyclohexyl]äthyl]-3,4-difluorbenzol;
4-Fluorbenzoesäure-trans-4-[trans-4-(4Z-hexenyl)cyclohexyl]cyclohexylester;
3,4-Difluorbenzoesäure-trans-4-[trans-4-(4Z-hexenyl)cyclohexyl]cyclohexylester;
trans-4-[trans-4-(4Z-Hexenyl)cyclohexyl]cyclohexyl-4-fluorbenzyläther;
trans-4-[trans-4-(4Z-Hexenyl)cyclohexyl]cyclohexyl-3,4-difluorbenzyläther.

Durch Weglassen der Stufen b) und c) können in analoger Weise auch die in Beispiel 1 genannten 3E-Alkenylverbindungen sowie die folgenden Verbindungen hergestellt werden:

1-[trans-4-[trans-4-(3-Butenyl)cyclohexyl]cyclohexyl-3,4-difluorbenzol;
1-[trans-4-[trans-4-(3-Butenyl)cyclohexyl]cyclohexyl-3-chlor-4-fluorbenzol;

1-[trans-4-[trans-4-(3-Butenyl)cyclohexyl]cyclohexyl-4-chlorbenzol;
1-[trans-4-[trans-4-(3-Butenyl)cyclohexyl]cyclohexyl-3-fluor-4-chlorbenzol;
1-[trans-4-[2-(trans-4-(3-Butenyl)cyclohexyl)äthyl]cyclohexyl]-3,4-difluorbenzol;
1-[trans-4-[2-(trans-4-(3-Butenyl)cyclohexyl)äthyl]cyclohexyl]-3-chlor-4-fluorbenzol;
1-[trans-4-[2-(trans-4-(3-Butenyl)cyclohexyl)äthyl]cyclohexyl]-4-chlorbenzol;
1-[trans-4-[2-(trans-4-(3-Butenyl)cyclohexyl)äthyl]cyclohexyl]-3-fluor-4-chlorbenzol;
1-[[trans-4-(trans-4-(3-Butenyl)cyclohexyl)cyclohexyl]methoxy]-4-fluorbenzol Smp. (C-N) 82,2 °C, Klp. (N-I) 131,7 °C;
1-[[trans-4-(trans-4-(3-Butenyl)cyclohexyl)cyclohexyl]methoxy]-3,4-difluorbenzol;
1-[[trans-4-(trans-4-(3-Butenyl)cyclohexyl)cyclohexyl]methoxy]-3-chlor-4-fluorbenzol;
1-[[trans-4-(trans-4-(3-Butenyl)cyclohexyl)cyclohexyl]methoxy]-4-chlorbenzol;
1-[[trans-4-(trans-4-(3-Butenyl)cyclohexyl)cyclohexyl]methoxy]-3-fluor-4-chlorbenzol;
1-[2-[trans-4-(trans-4-(3-Butenyl)cyclohexyl)cyclohexyl]äthyl]-3,4-difluorbenzol;
1-[2-[trans-4-(trans-4-(3-Butenyl)cyclohexyl)cyclohexyl]äthyl]-3-chlor-4-fluorbenzol;
1-[2-[trans-4-(trans-4-(3-Butenyl)cyclohexyl)cyclohexyl]äthyl]-4-chlorbenzol;
1-[2-[trans-4-(trans-4-(3-Butenyl)cyclohexyl)cyclohexyl]äthyl]-3-fluor-4-chlorbenzol;
3,4-Difluorbenzoesäure-trans-4-[trans-4-(3-butenyl)cyclohexyl]cyclohexylester;
3-Chlor-4-fluorbenzoesäure-trans-4-[trans-4-(3-butenyl)cyclohexyl]cyclohexylester;
4-Chlorbenzoesäure-trans-4-[trans-4-(3-butenyl)cyclohexyl]cyclohexylester;
3-Fluor-4-chlorbenzoesäure-trans-4-[trans-4-(3-butenyl)cyclohexyl]cyclohexylester;
trans-4-[trans-4-(3-Butenyl)cyclohexyl]cyclohexyl-4-fluorbenzyläther, Smp. (C-N) 37,7 °C, Klp. (N-I) 92,8 °C;
trans-4-[trans-4-(3-Butenyl)cyclohexyl]cyclohexyl-3,4-difluorbenzyläther;
trans-4-[trans-4-(3-Butenyl)cyclohexyl]cyclohexyl-3-chlor-4-fluorbenzyläther;
trans-4-[trans-4-(3-Butenyl)cyclohexyl]cyclohexyl-4-chlorbenzyläther;
trans-4-[trans-4-(3-Butenyl)cyclohexyl]cyclohexyl-3-fluor-4-chlorbenzyläther;
1-[trans-4-[trans-4-(3E-Pentenyl)cyclohexyl]cyclohexyl]-3,4-difluorbenzol;
1-[trans-4-[2-(trans-4-(3E-Pentenyl)cyclohexyl)äthyl]cyclohexyl]-3,4-difluorbenzol;
1-[[trans-4-(trans-4-(3E-Pentenyl)cyclohexyl)cyclohexyl]methoxy]-4-fluorbenzol;
1-[[trans-4-(trans-4-(3E-Pentenyl)cyclohexyl)cyclohexyl]methoxy]-3,4-difluorbenzol;
1-[2-[trans-4-(trans-4-(3E-Pentenyl)cyclohexyl)cyclohexyl]äthyl]-3,4-difluorbenzol;
trans-4-[trans-4-(3E-Pentenyl)cyclohexyl]cyclohexyl-4-fluorbenzyläther;
trans-4-[trans-4-(3E-pentenyl)cyclohexyl]cyclohexyl-3,4-difluorbenzyläther;
3,4-Difluorbenzoesäure-trans-4-[trans-4-(3E-pentenyl)cyclohexyl]cyclohexylester;
trans-4-[trans-4-(3E-Pentenyl)cyclohexyl]cyclohexyl-3,4-difluorbenzyläther.

Beispiel 10

a) Eine Suspension von 1,20 g Lithiumaluminiumhydrid in 50 ml Diäthyläther wurde innert 15 Minuten tropfenweise mit einer Lösung von 6,67 g trans-4-(4-Fluorphenyl)cyclohexancarbonsäure in 200 ml Diäthyläther und 40 ml Tetrahydofuran versetzt. Das Reaktionsgemisch wurde 2 Stunden bei Raumtemperatur gerührt, dann tropfenweise mit 4 ml Aceton und 14 ml Wasser versetzt und mit 3N Salzsäure sauer (pH ca. 1) gestellt. Die wässrige Phase wurde abgetrennt. Die organische Phase wurde mit 30 ml Wasser, zweimal mit je 20 ml halbgesättigter Sodalösung und dreimal mit je 30 ml Wasser gewaschen, über Natriumsulfat getrocknet und eingedampft. Hierbei wurden 6,08 g rohes [trans-4-(4-Fluorphenyl)-cyclohexyl]methanol mit Smp. 65-70 °C erhalten.

b) Eine Suspension von 11,35 g Pyridiniumchlorochromat in 80 ml Dichlormethan wurde tropfenweise mit einer Lösung von 6,06 g [trans-4-(4-Fluorphenyl)cyclohexyl]methanol in 18 ml Diäthyläther versetzt. Das Reaktionsgemisch wurde 4 Stunden gerührt, dann mit 50 ml Diäthyläther verdünnt, vom schwarzen Rückstand dekantiert und filtriert. Einengen des Filtrates ergb 4,86 g trans-4-(4-Fluorphenyl)-cyclohexancarboxaldehyd als gelbliches Oel, das bei Raumtemperatur erstarrte; Smp. <30 °C.

Beispiel 11

In einem Sulfierkolben mit absteigendem Kühler wurde eine Lösung von 0,98 g 2-(4-Pentenyl)-1,3-propandiol und 1,34 g trans-4-(4-Fluorphenyl)cyclohexancarboxaldehyd in 40 ml Toluol mit 4 Tropfen 10-prozentiger Schwefelsäure versetzt. Das Gemisch wurde 1 Stunde gekocht, wobei das abdestillierte feuchte Lösungsmittel durch Zutropfen von frischem Toluol ersetzt wurde. Anschliessend wurde das Reaktionsgemisch mit Triäthylamin (ca. 8 Tropfen) neutralisiert, zweimal mit je 15 ml Wasser gewaschen, über Natriumsulfat getrocknet, filtriert und eingeengt. Chromatographische Reinigung des Rückstandes (2,22 g) an Kieselgel mit Hexan/Aethylacetat (Vol. 39:1) und mehrmalige Umkristallisation aus Aethanol ergab schliesslich 0,44 g reines trans-2-[trans-4-(4-Fluorphenyl)cyclohexyl]-5-(4-Pentenyl)-m-dioxan; Smp. (C-$S_B$) 68,5 °C, Phasenübergang ($S_B$-N) 77 °C, Klp. (N-I) 96,5 °C.

In analoger Weise können folgende Verbindungen hergestellt werden:
trans-2-[trans-4-(3,4-Difluorphenyl)cyclohexyl]-5-(4-pentenyl)-m-dioxan;
trans-2-[trans-4-(4-Chlorphenyl)cyclohexyl]-5-(4-pentenyl)-m-dioxan;
trans-2-[trans-4-(3,4-Difluorphenyl)cyclohexyl]-5-(3-butenyl)-m-dioxan;
trans-2-[trans-4-(4-Chlorphenyl)cyclohexyl]-5-(3-butenyl)-m-dioxan;
trans-2-[trans-4-[2-(4-Fluorphenyl)äthyl]cyclohexyl]-5-(4-pentenyl)-m-dioxan;
trans-2-[trans-4-[2-(3,4-Difluorphenyl)äthyl]cyclohexyl]-5-(4-pentenyl)-m-dioxan;
trans-2-[trans-4-[2-(4-Chlorphenyl)äthyl]cyclohexyl]-5-(4-pentenyl)-m-dioxan;
trans-2-[trans-4-[2-(3,4-Difluorphenyl)äthyl]cyclohexyl]-5-(3-butenyl)-m-dioxan;
trans-2-[trans-4-[2-(4-Chlorphenyl)äthyl]cyclohexyl]-5-(3-butenyl)-m-dioxan;
trans-2-(4-Fluorphenyl)-5-(4-pentenyl)-m-dioxan, Smp. (C-I) 18,7 °C;
trans-2-(3,4-Difluorphenyl)-5-(4-pentenyl)-m-dioxan;
trans-2-(4-Chlorphenyl)-5-(4-pentenyl)-m-dioxan;
trans-2-(3,4-Difluorphenyl)-5-(3-butenyl)-m-dioxan;
trans-2-(4-Chlorphenyl)-5-(3-butenyl)-m-dioxan;
trans-2-[2-(4-Fluorphenyl)äthyl]-5-(4-pentenyl)-m-dioxan;
trans-2-[2-(3,4-Difluorphenyl)äthyl]-5-(4-pentenyl)-m-dioxan;
trans-2-[2-(4-Chlorphenyl)äthyl]-5-(4-pentenyl)-m-dioxan;
trans-2-[2-(3,4-Difluorphenyl)äthyl]-5-(3-butenyl)-m-dioxan;
trans-2-[2-(4-Chlorphenyl)äthyl]-5-(3-butenyl)-m-dioxan.

Beispiel 12

Ein Gemisch von 3,6 g 7-Dimethoxy-6-(dimethoxymethyl)-1-hepten. 75 mg p-Toluolsulfonsäure-monohydrat und 0,35 ml Wasser wurde unter Rühren und Stickstoffatmosphäre in einem Oelbad von 110 °C 2 Stunden zu leichtem Sieden erhitzt. Danach wurde das Gemisch mit 0,5 g Natriumhydrogencarbonat versetzt, 10 Minuten ohne Oelbad gerührt und dann filtriert (Nachwaschen mit wenig Methanol). Das Filtrat wurde zu einer Lösung von 2,9 g 4-Fluorbenzamidin-hydrochlorid in 60 ml Methanol gegeben. Anschliessend wurde das Reaktionsgemisch tropfenweise mit einer Natriummethylat-Lösung (hergestellt aus 0,6 g Natrium und 15 ml Methanol) versetzt, über Nacht bei Raumtemperatur gerührt, dann mit konzentrierter Salzsäue auf pH 5 gestellt und eingedampft. Der Rückstand wurde mit Wasser versetzt und mit Diäthyläther extrahiert. Die organische Phase wurde mit Wasser gewaschen, über Natriumsulfat getrocknet, filtriert und eingedampft. Chromatographische Reinigung des Rohproduktes an Kieselgel mit Hexan/Aethylacetat (Vol. 19:1) und Umkristallisation aus Hexan ergab 1,0 g reines 2-(4-Fluorphenyl)-5-(4-pentenyl)pyrimidin mit Smp. 21,8 °C.

In analoger Weise können folgende Verbindungen hergestellt werden:
2-(3,4-Difluorphenyl)-5-(4-pentenyl)pyrimidin;
2-(4-Chlorphenyl)-5-(4-pentenyl)pyrimidin;
2-(3,4-Difluorphenyl)-5-(3-butenyl)pyrimidin;
2-(4-Chlorphenyl)-5-(3-butenyl)pyrimidin;
2-(4-Fluorphenyl)-5-[trans-4-(4-pentenyl)cyclohexyl]pyrimidin;
2-(3,4-Difluorphenyl)-5-[trans-4-(4-pentenyl)cyclohexyl]pyrimidin;
2-(4-Chlorphenyl)-5-[trans-4-(4-pentenyl)cyclohexyl]pyrimidin;
2-(3,4-Difluorphenyl)-5-[trans-4-(3-butenyl)cyclohexyl]pyrimidin;
2-(4-Chlorphenyl)-5-[trans-4-(3-butenyl)cyclohexyl]pyrimidin;
2-(4′-Fluor-4-biphenylyl)-5-(4-pentenyl)pyrimidin;
2-(3′,4′-Difluor-4-biphenylyl)-5-(4-pentenyl)pyrimidin;
2-(4′-Chlor-4-biphenylyl)-5-(4-pentenyl)pyrimidin;
2-(3′,4′-Difluor-4-biphenylyl)-5-(3-butenyl)pyrimidin;
2-(3′-Chlor-4-biphenylyl)-5-(3-butenyl)pyrimidin.

Beispiel 13

Aus 4-(trans-4-Pentylcyclohexyl)benzonitril und einer Verbindung der Formel I wurden die nachstehend aufgeführten binären Gemische hergestellt. Die elektro-optischen Daten wurden in einer TN-Zelle mit einem Plattenabstand von 8 µm bei 22 °C gemessen. Die entsprechenden Werte für 4-(trans-4-Pentylcyclohexyl)-benzonitril betragen: $V_{10}$ = 1,62V, $t_{on}$ = 30 ms, Δn = 0,120; Klp. (N-I) 54,6 °C.

Mischung 1

90 Gew.-% 4-(trans-4-Pentylcyclohexyl)benzonitril,
10 Gew.-% 4-(3-Butenyloxy)benzoesäure-4-fluorphenylester;
$V_{10}$ = 1,29V, $t_{on}$ = 31 ms, Δn = 0,117.

Mischung 2

90 Gew.-% 4-(trans-4-Pentylcyclohexyl)benzonitril,
10 Gew.-% trans-4-(4-Pentenyl)cyclohexancarbonsäure-4-fluorphenylester;
$V_{10}$ = 1.17V, $t_{on}$ = 22 ms, Δn = 0,108.

Mischung 3

70 Gew.-% 4-(trans-4-Pentylcyclohexyl)benzonitril,
30 Gew.-% trans-4-[trans-4-(4-Pentenyl)cyclohexyl]cyclohexancarbonsäure-4-fluorphenylester;
$V_{10}$ = 1,55V, $t_{on}$ = 44 ms, Δn = 0,110; Klp. (N-I) 75,3 °C.

Mischung 4

90 Gew.-% 4-(trans-4-Pentylcyclohexyl)benzonitril,
10 Gew.-% 4-(4-Pentenyl)benzoesäure-4-fluorphenylester;
$V_{10}$ = 1,42V, $t_{on}$ = 26 ms, Δn = 0,117; Klp. (N-I) 50,3 °C.

Mischung 5

90 Gew.-% 4-(trans-4-Pentylcyclohexyl)benzonitril,
10 Gew.-% 4-(3-Butenyloxy)benzoesäure-3,4-difluorphenylester;
$V_{10}$ = 1,23V, $t_{on}$ = 36 ms, Δn = 0,116; Klp. (N-I) 44,5 °C.

Mischung 6

90 Gew.-% 4-(trans-4-Pentylcyclohexyl)benzonitril,
10 Gew.-% 1-[trans-4-[trans-4-(4-Pentenyl)cyclohexyl]cyclohexyl]-4-fluorbenzol;
$V_{10}$ = 1,47V, $t_{on}$ = 25 ms, Δn = 0,112; Klp. (N-I) 59,1 °C.

Mischung 7

90 Gew.-% 4-(trans-4-Pentylcyclohexyl)benzonitril,
10 Gew.-% trans-2-[trans-4-(4-Fluorphenyl)cyclohexyl]-5-(4-pentenyl)-m-dioxan;
$V_{10}$ = 1,34V, $t_{on}$ = 31 ms, Δn = 0,116; Klp. (N-I) 54,0 °C.

Beispiel 14

Die folgenden Mischungen wurden hergestellt. Die elektro-optischen Daten wurden in einer TN-Zelle mit einem Plattenabstand von 6 µm bei 22 °C gemessen, sofern nicht anders angegeben.

Mischung A

15 Gew.-% trans-4-[trans-4-(3-Butenyl)cyclohexyl]cyclohexancarbonitril,
12 Gew.-% trans-4-[trans-4-(3E-Pentenyl)cyclohexyl]cyclohexancarbonitril,
10 Gew.-% trans-4-[trans-4-(4-Pentenyl)cyclohexyl]cyclohexancarbonitril,
10 Gew.-% 4-[trans-4-(1E-Propenyl)cyclohexyl]benzonitril,
20 Gew.-% trans-4-Allyloxy-1-(trans-4-propylcyclohexyl)cyclohexan,
15 Gew.-% 4-Aethoxy-1-[trans-4-(3E-pentenyl)cyclohexyl]benzol,
10 Gew.-% trans-4-[trans-4-(3-Butenyl)cyclohexyl]cyclohexancarbonsäure-4-fluorphenylester,
8 Gew.-% trans-4-[trans-4-(4-Pentenyl)cyclohexyl]cyclohexancarbonsäure-4-fluorphenylester;
Smp. <-30 °C, Klp. (N-I) 71 °C; $V_{10}$ = 2,10V, p = 0,204,
$t_{on}$ = 19 ms, $t_{off}$ = 31 ms, Δn, = 0,084.

Mischung B

15 Gew.-% trans-4-[trans-4-(3-Butenyl)cyclohexyl]cyclohexancarbonitril,
12 Gew.-% trans-4-[trans-4-(3E-Pentenyl)cyclohexyl]cyclohexancarbonitril,
10 Gew.-% trans-4-[trans-4-(4-Pentenyl)cyclohexyl]cyclohexancarbonitril,
33 Gew.-% trans-4-Allyloxy-1-(trans-4-propylcyclohexyl)cyclohexan,
20 Gew.-% trans-4-[trans-4-(3-Butenyl)cyclohexyl]cyclohexancarbonsäure-4-fluorphenylester.
10 Gew.-% trans-4-[trans-4-(4-Pentenyl)cyclohexyl]cyclohexancarbonsäure-4-fluorphenylester;
Smp. <-20 °C, Klp. (N-I) 81 °C; $V_{10}$ = 2,41V, p = 0,211, $t_{on}$ = 24 ms, $t_{off}$ = 37 ms, Δn = 0,067.

Mischung C

10 Gew.-% 4-[trans-4-(1E-Propenyl)cyclohexyl]benzonitril,
15 " 4-Aethoxy-1-[trans-4-(3E-Pentenyl)cyclohexyl]benzol,
12 " trans-4-[trans-4-(3-Butenyl)cyclohexyl]cyclohexancarbonitril,
10 " trans-4-[trans-4-(3E-Pentenyl)cyclohexyl]cyclohexancarbonitril,
8 " trans-4-[trans-4-(4-Pentenyl)cyclohexyl]cyclohexancarbonitril,
15 " trans-4-(3-Butenyl)-1-(trans-4-äthoxycyclohexyl)-cyclohexan,
8 " 1-[2-(trans-4-Butylcyclohexyl)äthyl]-4-(trans-4-pentylcyclohexyl)benzol,
8 " trans-4-[trans-4-(3-Butenyl)cyclohexyl]cyclohexancarbonsäure-4-fluorphenylester,
8 " trans-4-[trans-4-(3E-Pentenyl)cyclohexyl]cyclohexancarbonsäure-4-fluorphenylester,
6 " trans-4-[trans-4-(4-Pentenyl)cyclohexyl]cyclohexancarbonsäure-4-fluorphenylester;

Smp. <-30 °C, Klp. 84 °C, nematisch; $V_{10}$ = 2,35V; $t_{on}$ (22 °C) = 15 ms, $t_{on}$ (-20 °C) = 309 ms, $t_{off}$ (22 °C) = 27 ms, $t_{off}$ (-20 °C) = 420 ms; Δn = 0,088.

Mischung D

| | | |
|---|---|---|
| 10 | Gew.-% | 4-[trans-4-(1E-Propenyl)cyclohexyl]benzonitril, |
| 6 | " | 4-[trans-4-(3E-Propenyl)cyclohexyl]benzonitril, |
| 12 | " | 4-Aethoxy-1-[trans-4-(3E-pentenyl)cyclohexyl]benzol, |
| 5 | Gew.-% | trans-4-[trans-4-(3-Butenyl)cyclohexyl]cyclohexan-carbonitril, |
| 7 | " | trans-4-[trans-4-(3E-Pentenyl)cyclohexyl]cyclohexan-carbonitril, |
| 12 | " | trans-4-Allyloxy-1-(trans-4-propylcyclohexyl)cyclo-hexan, |
| 10 | " | trans-4-(3E-Pentenyl)-1-(trans-4-äthoxycyclohexyl)-cyclohexan, |
| 6 | " | 4-[trans-4-(3E-Pentenyl)cyclohexyl]-4'-propyl-biphenyl, |
| 6 | " | trans-4-Propylcyclohexancarbonsäure-4-[trans-4-(3E-pentenyl)cyclohexyl]phenylester, |
| 10 | " | 1-[2-(trans-4-Butylcyclohexyl)äthyl]-4-(trans-4-pentylcyclohexyl)benzol, |
| 4 | " | 4-[2-(trans-4-Butylcyclohexyl)äthyl]-4'-[trans-4-(3E-pentenyl)cyclohexyl]biphenyl, |
| 8 | " | trans-4-[trans-4-(3-Butenyl)cyclohexyl]cyclohexan-carbonsäure-4-fluorphenylester, |
| 4 | " | trans-4-[trans-4-(3E-Pentenyl)cyclohexyl]cyclohexan-carbonsäure-4-fluorphenylester; |

Smp. <-30 °C, Klp. 100 °C, nematisch; $V_{10}$ = 2,82V, $t_{on}$ (22 °C) = 12 ms, $t_{on}$ = (-20 °C) = 220 ms, $t_{off}$ - (22 °C) = 21 ms, $t_{off}$ (-20 °C) = 330 ms; $\Delta n$ = 0,105.

Mischung E

| | | |
|---|---|---|
| 9,40 Gew.-% | | 4-[trans-4-(1E-Propenyl)cyclohexyl]benzonitril, |
| 5,64 | " | 4-[trans-4-(3E-Pentenyl)cyclohexyl]benzonitril, |
| 11,28 | " | 4-Aethoxy-1-[trans-4-(3E-pentenyl)cyclohexyl]-benzol, |
| 4,70 | " | trans-4-[trans-4-(3-Butenyl)cyclohexyl]cyclohexancarbonitril, |
| 6,58 | " | trans-4-[trans-4-(3E-Pentenyl)cyclohexyl]cyclohexancarbonitril, |
| 11,28 | " | trans-4-Allyloxy-1-(trans-4-propylcyclohexyl) cyclohexan, |
| 9,40 Gew.-% | | trans-4-(3E-Pentenyl)-1-(trans-4-äthoxycyclohexyl)cyclohexan, |
| 6,00 | " | 2-Cyano-5-(trans-4-butylcyclohexyl)pyrimidin, |
| 5,64 | " | trans-4-Propylcyclohexancarbonsäure-4-[trans-4-(3E-pentenyl)cyclohexyl]phenylester, |
| 5,64 | " | 4-[trans-4-(3E-Pentenyl)cyclohexyl]-4'-propylbiphenyl, |
| 9,40 | " | 1-[2-(trans-4-Butylcyclohexyl)äthyl]-4-(trans-4-pentylcyclohexyl)benzol, |
| 3,76 | " | 4-[2-(trans-4-Butylcyclohexyl)äthyl]-4'-[trans-4-(3E-pentenyl)cyclohexyl]biphenyl, |
| 7,52 | " | trans-4-[trans-4-(3-Butenyl)cyclohexyl]cyclohexancarbonsäure-4-fluorphenylester, |
| 3,76 | " | trans-4-[trans-4-(3E-Pentenyl)cyclohexyl]cyclohexancarbonsäure-4-fluorphenylester; |

Smp. <-30 °C, Klp. 95 °C, nematisch; $V_{10}$ = 2,35V, $t_{on}$ (22 °C) = 16 ms, $t_{on}$ = (-20 °C) = 300 ms, $t_{off}$ - (22 °C) = 24 ms, $t_{off}$ (-20 °C) = 350 ms; Δn = 0,104.

Mischung F

| | | |
|---|---|---|
| 10 | Gew.-% | 4-[trans-4-(1E-Propenyl)cyclohexyl]benzonitril, |
| 12 | " | 4-(2E-Butenyloxy)-1-(trans-4-propylcyclohexyl)-benzol, |
| 5 | " | trans-4-[trans-4-(3-Butenyl)cyclohexyl]cyclohexancarbonitril, |
| 7 | " | trans-4-[trans-4-(3E-Pentenyl)cyclohexyl]cyclohexancarbonitril, |
| 6 | " | trans-4-[trans-4-(4-Pentenyl)cyclohexyl]cyclohexancarbonitril, |
| 12 | " | trans-4-Allyloxy-1-(trans-4-propylcyclohexyl)cyclohexan, |
| 10 | " | trans-4-(3E-Pentenyl)-1-(trans-4-äthoxycyclohexyl)-cyclohexan, |
| 8 | " | trans-4-Propylcyclohexancarbonsäure-4-[trans-4-(3E-pentenyl)cyclohexyl]phenylester, |

| | | |
|---|---|---|
| 10 | Gew.-% | 1-[2-(trans-4-Butylcyclohexyl)äthyl]-4-(trans-4-pentylcyclohexyl)benzol, |
| 5 | " | 4-[2-(trans-4-Butylcyclohexyl)äthyl]-4-(trans-4-pentylcyclohexyl)biphenyl, |
| 3 | " | 5-(trans-4-Pentylcyclohexyl)-2-[4-(trans-4-propylcyclohexyl)phenyl]pyrimidin, |
| 4 | " | trans-4-[trans-4-(3-Butenyl)cyclohexyl]cyclohexancarbonsäure-4-fluorphenylester, |
| 8 | " | trans-4-[trans-4-(3E-Pentenyl)cyclohexyl]cyclohexancarbonsäure-4-fluorphenylester; |

Smp. <-30 °C, Klp. 109 °C, nematisch; $V_{10}$ = 2,84V, $t_{on}$ (22 °C) = 16 ms, $t_{on}$ = (-20 °C) = 336 ms, $t_{off}$ - (22 °C) = 26 ms, $t_{off}$ (-20 °C) = 370 ms; Δn = 0,096.

Mischung G

| | | |
|---|---|---|
| 10 | Gew.-% | 4-(trans-4-Propylcyclohexyl)benzonitril, |
| 8 | " | 4-[trans-4-(1E-Propenyl)cyclohexyl]benzonitril, |
| 10 | " | trans-4-(trans-4-Vinylcyclohexyl)cyclohexancarbonitril, |
| 8 | " | trans-4-[trans-4-(3E-Pentenyl)cyclohexyl]cyclohexancarbonitril, |
| 10 | " | trans-4-Aethoxy-1-[trans-4-(3E-pentenyl)cyclohexyl]cyclohexan, |
| 8 | " | trans-4-Aethoxy-1-[trans-4-(4-pentenyl)cyclohexyl]-cyclohexan, |
| 8 | " | 2-Cyano-5-(trans-4-butylcyclohexyl)pyrimidin, |
| 3 | " | 2-Cyano-5-(trans-4-pentylcyclohexyl)pyrimidin, |
| 7 | " | 4-(2E-Butenyloxy)-1-[trans-4-(trans-4-propylcyclohexyl)cyclohexyl]benzol, |
| 5 | " | trans-4-[5-(trans-4-Butylcyclohexyl)-2-pyrimidinyl]-cyclohexancarbonitril, |
| 8 | " | trans-4-Propylcyclohexancarbonsäure-4-[trans-4-(3E-pentenyl)cyclohexyl]phenylester, |
| 7 | | 1-[trans-4-(trans-4-Propylcyclohexyl)cyclohexyl-4-fluorbenzol, |
| 5 | Gew.-% | trans-4-[trans-4-(3-Butenyl)cyclohexyl]cyclohexancarbonsäure-4-fluorphenylester, |
| 7 | " | trans-4-[trans-4-(4-Pentenyl)cyclohexyl]cyclohexancarbonsäure-4-fluorphenylester; |

Smp. <-30 °C, Klp. 92 °C, nematisch; $V_{10}$ = 1,71V, $t_{on}$ (22 °C) = 19 ms, $t_{on}$ = (-20 °C) = 530 ms, $t_{off}$ - (22 °C) = 34 ms, $t_{off}$ (-20 °C) = 772 ms; $\Delta n$ = 0,094.

Mischung H

| | | |
|---|---|---|
| 7 | Gew.-% | 4-(trans-4-Propylcyclohexyl)benzonitril, |
| 8 | " | 4-[trans-4-(1E-Propenyl)cyclohexyl]benzonitril, |
| 10 | " | trans-4-(trans-4-Vinylcyclohexyl)cyclohexan-carbonitril, |
| 10 | " | trans-4-Methoxy-1-[trans-4-(3E-pentenyl)cyclo-hexyl]cyclohexan, |
| 10 | " | trans-4-Aethoxy-1-[trans-4-(3-butenyl)cyclohexyl]-cyclohexan, |
| 12 | " | trans-4-Aethoxy-1-[trans-4-(3E-pentenyl)cyclohexyl]-cyclohexan, |
| 8 | " | 1-[2-(trans-4-Butylcyclohexyl)äthyl]-4-(trans-4-pentylcyclohexl)benzol, |
| 6 | " | trans-4-Propylcyclohexancarbonsäure-4-[trans-4-(3E-pentenyl)cyclohexyl]phenylester, |
| 8 | | 1-[trans-4-(trans-4-Propylcyclohexyl)cyclohexl]-4-fluorbenzol, |
| 6 | " | 1-[trans-4-(trans-4-Vinylcyclohexyl)cyclohexyl]-4-fluorbenzol, |
| 4 | " | 4-[2-(trans-4-Butylcyclohexyl)äthyl]-4'-[trans-4-(3E-pentenyl)cyclohexl]biphenyl, |
| 3 | " | 5-(trans-4-Pentylcyclohexyl)-2-[4-(trans-4-propyl-cyclohexyl)phenyl]pyrimidin, |
| 8 | " | trans-4-(4-Pentenyl)cyclohexancarbonsäure-4-fluor-phenylester; |

Smp. <-30 °C, Klp. 88 °C, nematisch; $V_{10}$ = 2,29V, $t_{on}$ (22 °C) = 12 ms, $t_{on}$ = (-20 °C) = 221 ms, $t_{off}$ - (22 °C) = 23 ms, $t_{off}$ (-20 °C) = 296 ms; $\Delta n$ = 0,080.

Mischung I

| | | |
|---|---|---|
| 7 | Gew.-% | 4-(trans-4-Propylcyclohexyl)benzonitril, |
| 8 | " | 4-(trans-4-(1E-Propenyl)cyclohexyl)benzonitril, |
| 10 | " | trans-4-(trans-4-Vinylcyclohexyl)cyclohexancarbonitril, |
| 10 | " | trans-4-Methoxy-1-[trans-4-(3E-pentenyl)cyclohexyl]cyclohexan, |
| 10 | " | trans-4-Aethoxy-1-[trans-4-(3-butenyl)cyclohexyl]-cyclohexan, |
| 12 | " | trans-4-Aethoxy-1-[trans-4-(3E-pentenyl)cyclohexyl]-cyclohexan, |
| 8 | " | 1-[2-(trans-4-Butylcyclohexyl)äthyl]-4-(trans-4-pentylcyclohexyl)benzol, |
| 6 | " | trans-4-Propylcyclohexancarbonsäure-4-[trans-4-(3E-pentenyl)cyclohexyl]phenylester, |
| 8 | " | 1-[trans-4-(trans-4-Propylcyclohexyl)cyclohexyl]-4-fluorbenzol, |
| 6 | " | 1-[trans-4-(trans-4-Vinylcyclohexyl)cyclohexyl]-4-fluorbenzol, |
| 4 | " | 4-[2-(trans-4-Butylcyclohexyl)äthyl]-4'-[trans-4-(3E-pentenyl)cyclohexyl]biphenyl, |
| 3 | " | 5-(trans-4-Pentylcyclohexyl)-2-[4-(trans-4-propylcyclohexyl)phenyl]pyrimidin, |
| 8 | " | trans-2-(4-Fluorphenyl)-5-(4-pentenyl)-m-dioxan; |

Smp. <-30 °C, Klp. 81 °C, nematisch; $V_{10}$ = 2,15V, $t_{on}$ (22 °C) = 14 ms, $t_{on}$ = (-20 °C) = 249 ms, $t_{off}$ - (22 °C) = 26 ms, $t_{off}$ (-20 °C) = 342 ms; Δn = 0,087.

Mischung J

10 Gew.-% 4-(trans-4-Propylcyclohexyl)benzonitril,
7 " 4-(trans-4-Vinylcyclohexyl)benzonitril,
8 " 4-[trans-4-(1E-Propenyl)cyclohexyl]benzonitril,
6 " 4-Aethoxy-1-(trans-4-propylcyclohexyl)benzol,
10 " trans-4-Methoxy-1-[trans-4-(3E-pentenyl)cyclohexyl]cyclohexan,

10 Gew.-% trans-4-Aethoxy-1-[trans-4-(3-butenyl)cyclohexyl]-cyclohexan,
12 " trans-4-Aethoxy-1-[trans-4-(3E-pentenyl)cyclohexyl]-cyclohexan,
8 " 4-(2E-Butenyloxy)-1-[trans-4-(trans-4-propylcyclohexyl)cyclohexyl]benzol,
8 " 1-[2-(trans-4-Butylcyclohexyl)äthyl]-4-(trans-4-pentylcyclohexyl)benzol,
6 " trans-4-Propylcyclohexancarbonsäure-4-[trans-4-(3E-pentenyl)cyclohexyl]phenylester,
4 " 4,4'-Bis-(trans-4-propylcyclohexyl)biphenyl,
3 " 5-(trans-4-Pentylcyclohexyl)-2-[4-(trans-4-propylcyclohexyl)phenyl]pyrimidin,
8 " trans-4-[trans-4-(4-Pentenyl)cyclohexyl]cyclohexancarbonsäure-4-fluorphenylester;

Smp. <-30 °C, Klp. 95 °C, nematisch; $V_{10}$ = 2,41V, $t_{on}$ (22 °C) = 12 ms, $t_{on}$ = (-20 °C) = 195 ms, $t_{off}$ - (22 °C) = 21 ms, $t_{off}$ (-20 °C) = 276 ms; $\Delta n$ = 0,101.

## Patentansprüche

**1.** Verbindungen der allgemeinen Formel

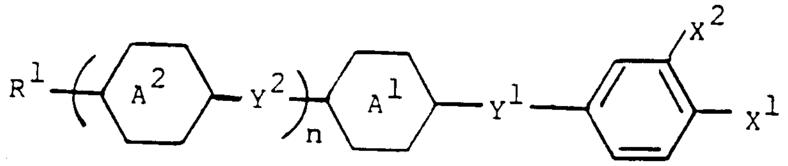

I

worin $X^1$ Fluor oder Chlor und $X^2$ Wasserstoff, Fluor oder Chlor bezeichnen; $R^1$ 3E-Alkenyl mit 4-15 Kohlenstoffatomen, 4-Alkenyl mit 5-15 Kohlenstoffatomen, 2E-Alkenyloxy mit 3-14 Kohlenstoffatomen oder 3-Alkenyloxy mit 4-14 Kohlenstoffatomen bedeutet; n für die Zahl 0 oder 1 steht; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes

oder unsubstituiertes 1,4-Phenylen, in welchem gegebenenfalls 1 oder 2 CH-Gruppen durch Stickstoff ersetzt sind, darstellen; mit der Massgabe, dass $X^2$ Fluor oder Chlor bezeichnet und/oder $Y^1$ oder $Y^2$ $-CH_2O-$ oder $-OCH_2$ bezeichnet, wenn zugleich $X^1$ Fluor und $R^1$ 3E-Alkenyl bedeuten.

**2.** Verbindungen nach Anspruch 1, dadurch gekennzeichnet, dass Ring $A^1$ für substituiertes oder unsubstituiertes trans-1,4-Cyclohexylen, für substituiertes oder unsubstituiertes 1,4-Phenylen oder für Pyridin-2,5-diyl, Pyrimidin-2,5-diyl oder Pyrazin-2,5-diyl steht.

**3.** Verbindungen nach Anspruch 1 oder 2, dadurch gekennzeichnet, dass Ring $A^2$ für substituiertes oder unsubstituiertes trans-1,4-Cyclohexylen, für substituiertes oder unsubstituiertes 1,4-Phenylen oder, wenn Ring $A^1$ substituiertes oder unsubstituiertes trans-1,4-Cyclohexylen oder substituiertes oder unsubstituiertes 1,4-Phenylen bedeutet, auch für Pyrimidin-2,5-diyl oder trans-m-Dioxan-2,5-diyl steht.

**4.** Verbindungen nach einem der Ansprüche 1 bis 3, dadurch gekennzeichnet, dass Ring $A^1$ trans-1,4-Cyclohexylen, 1,4-Phenylen, Pyridin-2,5-diyl, Pyrimidin-2,5-diyl oder Pyrazin-2,5-diyl bezeichnet und Ring $A^2$ trans-1,4-Cyclohexylen bedeutet.

**5.** Verbindungen nach Anspruch 1, gekennzeichnet durch die allgemeinen Formel

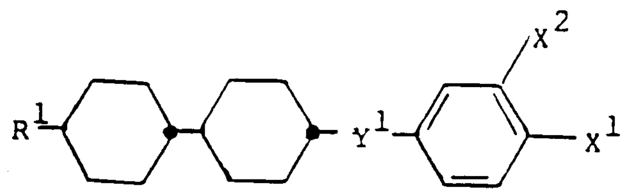

I-23

worin $R^1$, $X^1$, $X^2$ und $Y^1$ die in Anspruch 1 gegebenen Bedeutungen haben.

**6.** Verbindungen nach einem der Ansprüche 1 bis 5, dadurch gekennzeichnet, dass $X^1$ Fluor und $X^2$ Wasserstoff bedeuten, oder $X^1$ und $X^2$ Fluor bedeuten, oder $X^1$ Chlor und $X^2$ Wasserstoff bedeuten.

**7.** Verbindungen nach einem der Ansprüche 1 bis 6, dadurch gekennzeichnet, dass $R^1$ einen geradkettigen Rest bedeutet.

**8.** Flüssigkristallines Gemisch mit mindestens 2 Komponenten, dadurch gekennzeichnet, dass mindestens eine Komponente eine Verbindung gemäss Anspruch 1 ist und in einer Menge von etwa 1-50Gew.-% vorliegt.

**9.** Verwendung der Verbindungen der in Anspruch 1 definierten Formel I für elektro-optische Zwecke.

**Claims**

**1.** Compounds of the general formula

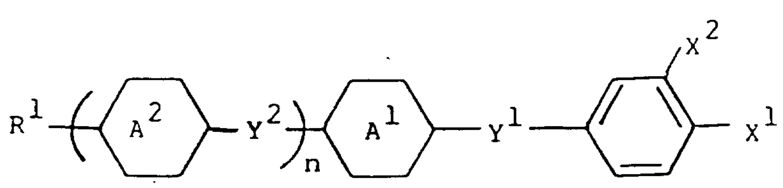

I

wherein $X^1$ denotes fluorine or chlorine and $X^2$ denotes hydrogen, fluorine or chlorine; $R^1$ signifies 3E-alkenyl having 4-15 carbon atoms, 4-alkenyl having 5-15 carbon atoms, 2E-alkenyloxy having 3-14 carbon atoms or 3-alkenyloxy having 4-14 carbon atoms; n stands for the number O or 1; one of the groups $Y^1$ and $Y^2$ signifies a single covalent bond, -COO-, -OOC-, $-CH_2CH_2-$, $-CH_2O-$ or $-OCH_2-$ and the other of the groups $Y^1$ and $Y^2$ signifies a single covalent bond; and rings $A^1$ and $A^2$ each individually

signify substituted or unsubstituted trans-1,4-cyclohexylene, in which optionally 2 non-adjacent $CH_2$ groups are replaced by oxygen, or substituted or unsubstituted 1,4-phenylene, in which optionally 1 CH group or 2 CH groups is/are replaced by nitrogen; with the proviso that $X^2$ denotes fluorine or chlorine and/or $Y^1$ or $Y^2$ denotes $-CH_2O-$ or $-OCH_2-$ when simultaneously $X^1$ signifies fluorine and $R^1$ signifies 3E-alkenyl.

2. Compounds according to claim 1, characterized in that ring $A^1$ stands for substituted or unsubstituted trans-1,4-cyclo-hexylene, for substituted or unsubstituted 1,4-phenylene or for pyridine-2,5-diyl, pyrimidine-2,5-diyl or pyrazine-2,5-diyl.

3. Compounds according to claim 1 or 2, characterized in that ring $A^2$ stands for substituted or unsubstituted trans-1,4-cyclohexylene, for substituted or unsubstituted 1,4-phenylene or, when ring $A^1$ signifies substituted or unsubstituted trans-1,4-cyclohexylene or substituted or unsubstituted 1,4-phenylene, also for pyrimidine-2,5-diyl or trans-m-dioxane-2,5-diyl.

4. Compounds according to any one of claims 1 to 3, characterized in that ring $A^1$ denotes trans-1,4-cyclohexylene, 1,4-phenylene, pyridine-2,5-diyl, pyrimidine-2,5-diyl or pyrazine-2,5-diyl and ring $A^2$ signifies trans-1,4-cyclohexylene.

5. Compounds according to claim 1, characterized by the general formula

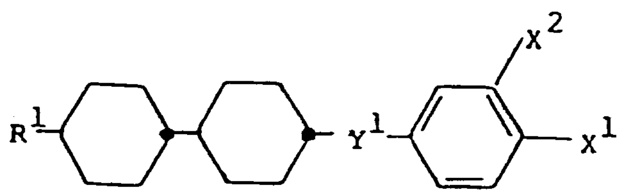

I-23

wherein $R^1$, $X^1$, $X^2$ and $Y^1$ have the significances given in claim 1.

6. Compounds according to any one of claims 1 to 5, characterized in that $X^1$ signifies fluorine and $X^2$ signifies hydrogen or $X^1$ and $X^2$ signify fluorine or $X^1$ signifies chlorine and $X^2$ signifies hydrogen.

7. Compounds according to any one of claims 1 to 6, characterized in that $R^1$ signifies a straight-chain residue.

8. A liquid crystalline mixture having at least 2 components, characterized in that at least one component is a compound in accordance with claim 1 and is present in an amount of about 1-50 wt.%.

9. The use of the compounds of formula I defined in claim 1 for electro-optical purposes.

## Revendications

1. Composés de formule générale

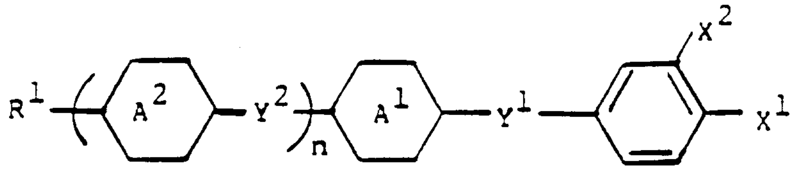

I

dans laquelle $X^1$ représente le fluor ou le chlore et $X^2$ l'hydrogène, le fluor ou le chlore ; $R^1$ représente un groupe 3E-alcényle en C4-C15, 4-alcényle en C5-C15, 2E-alcényloxy en C3-C14 ou 3-alcényloxy en

C4-C14 ; n est égal à 0 ou 1 ; l'un des symboles $Y^1$ et $Y^2$ représente une liaison covalente simple, -COO-, -OOC-, $-CH_2CH_2-$, $-CH_2O-$ ou $-OCH_2-$ et l'autre une liaison covalente simple ; et les cycles $A^1$ et $A^2$ consistent chacun, indépendamment l'un de l'autre, en un cycle trans-1,4-cyclohexylène substitué ou non substitué dans lequel le cas échéant deux groupes $CH_2$ non voisins sont remplacés par l'oxygène, ou en un groupe 1,4-phénylène substitué ou non substitué dans lequel, le cas échéant, un ou deux groupes CH sont remplacés par l'azote ; sous réserve que $X^2$ représente le fluor ou le chlore et/ou $Y^1$ ou $Y^2$ représente $-CH_2O$ou $-OCH_2$-lorsque, simultanément, $X^1$ représente le fluor et $R^1$ un groupe 3E-alcényle.

2. Composés selon la revendication 1, caractérisés en ce que le cycle $A^1$ est un cycle trans-1,4-cyclohexylène substitué ou non substitué, un groupe 1,4-phénylène substitué ou non substitué ou un groupe pyridine-2,5-diyle, pyrimidine-2,5-diyle ou pyrazine-2,5-diyle.

3. Composés selon la revendication 1 ou 2, caractérisés en ce que le cycle $A^2$ est un cycle trans-1,4-cyclohexylène substitué ou non substitué, un cycle 1,4-phénylène substitué ou non substitué, ou bien encore, lorsque le cycle $A^1$ est un cycle trans-1,4-cyclohexylène substitué ou non substitué ou un cycle 1,4-phénylène substitué ou non substitué, un cycle pyrimidine-2,5-diyle ou trans-m-dioxanne-2,5-diyle.

4. Composés selon l'une des revendications 1 à 3, caractérisés en ce que le cycle $A^1$ est un cycle trans-1,4-cyclohexylène, 1,4-phénylène, pyrimidine-2,5-diyle, pyrimidine-2,5-diyle ou pyrazine-2,5-diyle et le cycle $A^2$ un cycle trans-1,4-cyclo-hexylène.

5. Composés selon la revendication 1, caractérisés par la formule générale

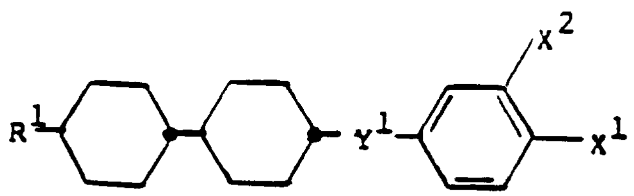

I-23

dans laquelle $R^1$, $X^1$, $X^2$ et $Y^1$ ont les significations indiquées dans la revendication 1.

6. Composés selon l'une des revendications 1 à 5, caractérisés en ce que $X^1$ représente le fluor et $X^2$ l'hydrogène, ou bien $X^1$ et $X^2$ représentent tous deux le fluor, ou bien $X^1$ représente le chlore et $X^2$ l'hydrogène.

7. Composés selon l'une des revendications 1 à 6, caractérisés en ce que $R^1$ est un radical à chaîne droite.

8. Mélange à cristaux liquides à au moins deux composants, caractérisé en ce qu'il contient au moins un composant consistant en un composé selon la revendication 1, en proportions d'environ 1 à 50% en poids.

9. Utilisation des composés de formule I de la revendication 1 dans des applications électro-optiques.